# EUROPEAN PATENT APPLICATION

(11) **EP 4 317 135 A1**
(43) Date of publication of application: **07.02.2024**
(21) Application number: 22778695.1
(22) Date of filing: 23.03.2022
(51) Int. Cl.: C07D 233/64, A61K 31/4174, A61P 25/00

(54) **5-BENZYLIMIDAZOLE COMPOUND AND PREPARATION METHOD THEREFOR AND USE THEREOF**

(30) Priority: 02.04.2021 CN 202110363563
(71) Applicant: West China Hospital, Sichuan University, Chengdu, Sichuan 610041 (CN)
(72) Inventor: YANG, Jun, Chengdu, Sichuan 610041 (CN); LIU, Jin, Chengdu, Sichuan 610041 (CN); ZHANG, Weiyi, Chengdu, Sichuan 610041 (CN)
(74) Representative: Altmann Stößel Dick Patentanwälte PartG mbB
(86) International application number: PCT/CN2022/082526
(87) International publication number: WO 2022/206512

(57) **Abstract**

Provided are a 5-benzylimidazole compound represented by formula I and a preparation method therefor and a use thereof. The compound of formula I and a pharmaceutically acceptable salt thereof can be decomposed in plasma to release the 5-benzylimidazole compound having a pharmacological effect, thereby exerting anesthetic and/or sedative-hypnotic effects in vivo. The compound of formula I has the characteristics of rapid onset, high potency, good circulation stability, and good safety, rapid injection does not cause severe blood pressure fluctuations, good anesthetic and/or sedative-hypnotic effects can also be achieved by oral administration, and the present invention has wide application prospects.

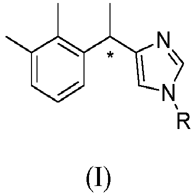

## Description

### Technical field

The present invention belongs to the biomedical field, and specifically relates to a 5-benzylimidazole compound, a preparation method, and uses thereof.

### Background technology

Dexmedetomidine hydrochloride is a commonly used ICU sedative drug in clinical practice, which has sedative, anti-anxiety, anti-sympathetic, and analgesic effects (Maud A. S. Weerinks, et al. Clin Pharmacokinet, 2017, 56: 893-913). Compared with other sedative medicaments, dexmedetomidine has a rapid action and mild respiratory suppression (Lobo FA, et al. Br J Anaesth. 2016; 116: 740-4), and its analgesic effect improves patient tolerance to intubate. Thereby, patients can have a sedative effect similar to natural sleep, who can be awakened and cooperate with medical activities at any time (Hall JE, et al. Aneth Analg. 2000; 90: 699-705). These advantages have led to the gradual attention of dexmedetomidine in postoperative sedation, ICU sedation, pediatric sedation, and other aspects. The structure of dexmedetomidine hydrochloride is as follows:

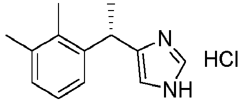

As an α2-adrenoceptor agonist, the therapeutic effect of dexmedetomidine depends on its stimulation on the central α2-adrenoceptors, which is manifested as sedative, analgesic, and the same (Miller's Anesthesia. 8th ed. Amsterdam: Elsevier; 2015, p. 854-9); and its side effects are mainly caused by its stimulation on the peripheral α2 adrenoceptors, which is mainly demonstrated by rapid elevation of blood pressure and bradycardia in feedback (Ebert TJ, et al. Anesthesiology. 2000; 93: 382-94). In addition, dexmedetomidine activates the α2 adrenoceptors in the central nervous system, which can produce anti-sympathetic effects and cause hypotension (MacMillan LB, et al. Science. 1996; 273: 801-3). Due to the fact that medicaments first enter the peripheral circulatory system and then diffuse into the central nervous system to play sedative and analgesic effects during clinical use, it is inevitable that there is a risk of excessive blood pressure increase in the early stage of administration, and subsequent bradycardia and hypotension in feedback, causing an unbearable blood pressure change for patients, that affects the safety of dexmedetomidine.

In order to address the above issues, in clinical practice, dexmedetomidine has to be slowly infused for 10-15 min during administration of the loading dose, followed by a maintenance dose (http://www.accessdata.fda.gov/drugsatfda_docs/label/1999/21038lbl.pdf), so that patients can tolerate the hemodynamic changes during infusion, which clearly increases the complexity of administration. The instability of circulation and the complicated administration mode limit the further clinical application of dexmedetomidine.

Therefore, there is an urgent need to develop a prodrug of dexmedetomidine hydrochloride, which should have good circulatory stability, high potency, and the ability to quickly and effectively produce sedative effects in the body, so as to simplify the administration method and expand the clinical application of dexmedetomidine.

### Summary of the invention

Aiming to solve the problem mentioned above, the present invention provides a prodrug molecule of dexmedetomidine, which is a 5-benzylimidazole compound, as well as a preparation method and use thereof.

The structures of these prodrug molecules are as represented by formula I: 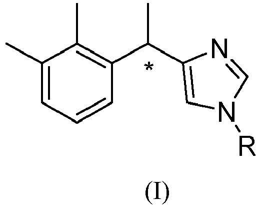 wherein, R is selected from the group consisting of 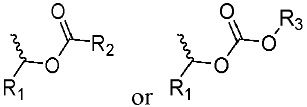 or 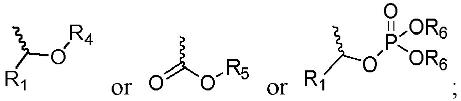
R₁ is selected from the group consisting of H, R^{a}, or R^{b}; R₆ is selected from the group consisting of H, monovalent alkali metal ion, R^{a}, or R^{b};
said R^{a} is substituted or unsubstituted C₁₋₄ alkyl; R^{b} is a chemically stable group formed by substituting some C atoms in R^{a} with heteroatoms O, S, and N;
R₂, R₃, and R₅ are each independently selected from R^{c} or R^{d};
said R^{c} is substituted or unsubstituted C₁₋₁₀ alkyl or 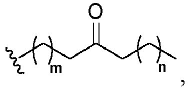 R^{d} is a chemically stable group formed by substituting some C atoms in R^{c} with heteroatoms O, S, and N; and R₅ is not tert-butyl; m is an integer of 0 to 3, and n is an integer of 0 to 3;
R₄ is a natural or unnatural amino acid fragment or 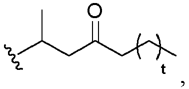 or a chemically stable group formed by substituting a part of the C atoms in the natural or unnatural amino acid fragment with heteroatoms O, S, N; t is an integer of 0 to 3;
the substituents in each group mentioned above are independently selected from the group consisting of halogen, hydroxyl, alkoxy, cyano, or nitro.

Further, R is selected from the group consisting of 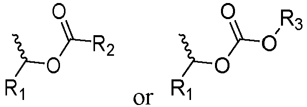 or 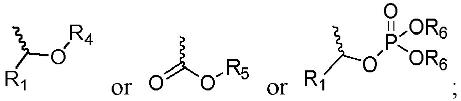
R₁ is selected from the group consisting of H, R^{a} or R^{b}; R₆ is selected from the group consisting of H, monovalent alkali metal ion, R^{a} or R^{b};
said R^{a} is substituted or unsubstituted C₁₋₄ alkyl; R^{b} is a chemically stable group formed by substituting some C atoms in R^{a} with heteroatoms O, S, and N;
R₂, R₃, and R₅ are each independently selected from R^{c} or R^{d};
said R^{c} is substituted or unsubstituted linear, branched or cyclic C₁₋₁₀ hydrocarbon groups; R^{d} is a chemically stable group formed by substituting some C atoms in R^{c} with heteroatoms O, S, and N; and R₅ is not tert-butyl;
R₄ is a natural or unnatural amino acid fragment, or a chemically stable group formed by substituting a part of the C atoms in the natural or unnatural amino acid fragment with heteroatoms O, S, N;
the substituents in each group mentioned above are independently selected from the group consisting of halogen, hydroxyl, alkoxy, cyano, or nitro.

More further, above R₁ is H or C₁₋₄ alkyl; R₂ and R₃ are each independently selected from linear, branched or cyclic C₁₋₁₀ hydrocarbon groups; R₅ is selected from linear, branched or cyclic C₁₋₁₀ hydrocarbon groups, and R₅ is not tert-butyl; R₄ is a natural or unnatural amino acid fragment; R₆ is H or monovalent alkali metal ion or C₁₋₄ alkyl;
alternatively, 0, 1 or more C atoms in R₁-R₅ are substituted with heteroatoms O, S, and N to form chemically stable groups; and/or 0, 1, or more H atoms in R₁-R₆ are substituted with halogen, hydroxyl, alkoxy, cyano, and/or nitro.

More further, R is 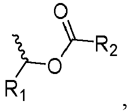 wherein R₁ is H, methyl or ethyl; R₂ is methyl, ethyl, propyl, butyl, isopropyl, cyclopropyl, cyclobutyl, methoxy, ethoxymethyl or vinyl; preferably, the compound has any one of the following structures:

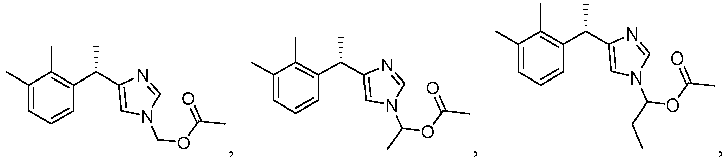

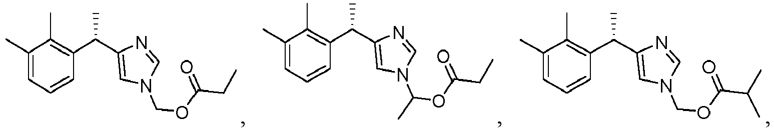

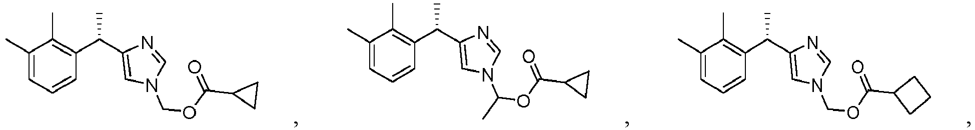

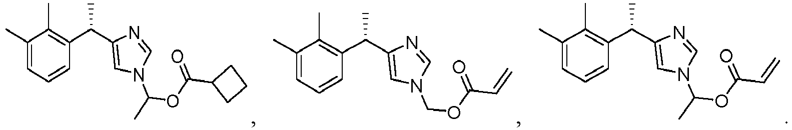

More further, R is 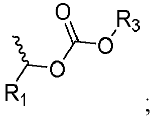 wherein R₁ is H, methyl or ethyl; R₃ is methyl, ethyl, propyl, butyl, isopropyl, cyclopropyl, cyclobutyl, methoxy, ethoxymethyl or vinyl; preferably, the compound has any one of the following structures:

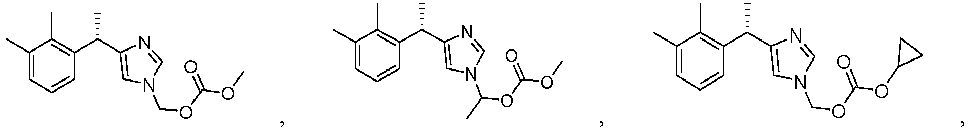

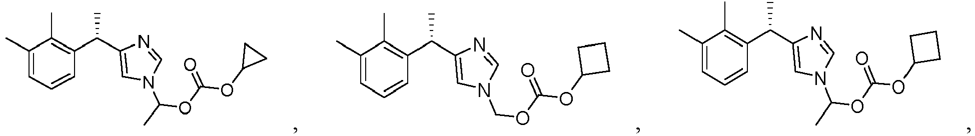

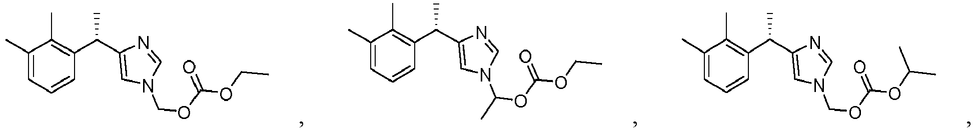

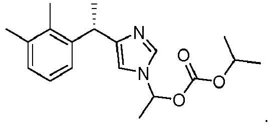

More further, R is 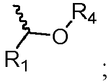 wherein, R₁ is H or methyl; R₄ is glycyl, sarcosinoyl, alanyl, valyl, leucyl, or prolinyl; preferably, the compound has any one of the following structures:

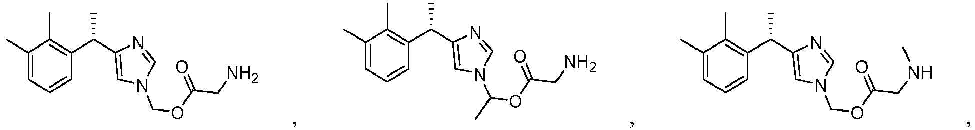

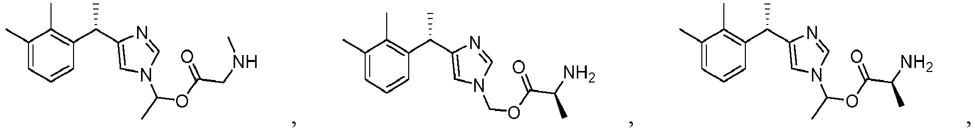

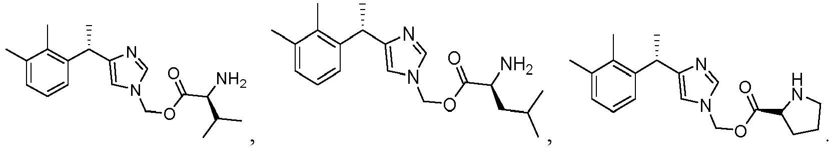

More further, R is 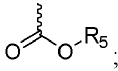 wherein, R₅ is methyl, ethyl, propyl, butyl, isopropyl, isobutyl, cyclopropyl or cyclobutyl; preferably, the compound has any one of the following structures:

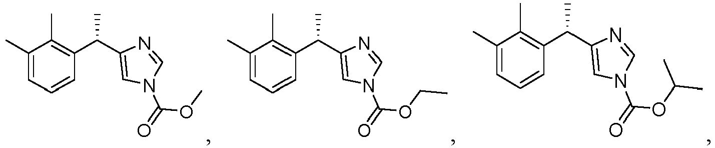

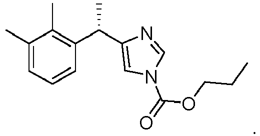

More further, R is 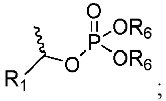 wherein R₆ is methyl, ethyl, propyl, butyl, isopropyl, tert-butyl, isobutyl, cyclopropyl or cyclobutyl; preferably, the compound has any one of the following structures:

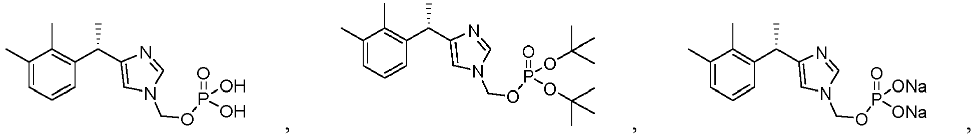

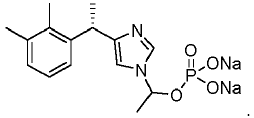

More further, the above pharmaceutically acceptable salts include acetate, adipate, alginate, 4-aminosalicylate, ascorbate, aspartate, glutamate, pyroglutamate, benzenesulfonate, benzoate, butyrate, camphorate, camphorsulfonate, carbonate, cinnamate, citrate, cyclamate, cyclopentylpropionate, decanoate, 2,2-dichloroacetate, gluconate, dodecylsulfate, ethane-1,2-disulfonate, ethanesulfonate, formate, fumarate, mucate, trifluoroacetate, gentisate, glucoheptonate, gluconate, glucuronate, glycerophosphate, hydroxy acetate, hemisulfate, heptanoate, caproate, hippurate, hydrochloride, hydrobromate, hydroiodate, 2-hydroxyethanesulfonate, isobutyrate, lactate, lactobionate, laurate, malate, maleate, malonate, mandelate, methanesulfonate, naphthalene-1,5-disulfonate, 2-naphthalenesulfonate, nicotinate, nitrate, octanoate, oleate, orotate, oxalate, 2-oxoglutarate, palmitate, pamoate, pectinate, persulfate, 3-phenylpropionate, phosphate, picrate, neovalerate, propionate, salicylate, sebacate, bisebacate, stearate, succinate, sulfate, tannate, tartrate, bitartrate, thiocyanate, toluenesulfonate or undecanoate, hydrosulfide, sodium salt or ammonium salt of the compounds.

The present invention also provides a preparation method for the compound mentioned above, which comprises the following steps:
(1) Compound **A** is dissolved in a solvent;
(2) Compound **B** and an acid-binding agent are added and allowed to react;

The reaction pathway is as follows: 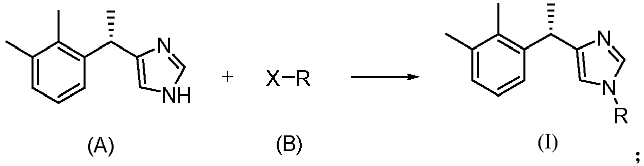 wherein, X is halogen, and preferably Cl;
R is selected from 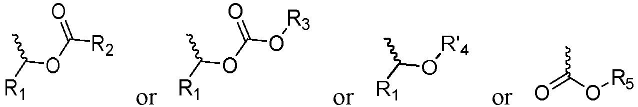 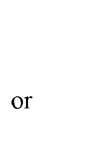 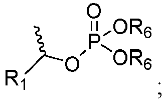
R₁ is H or C₁₋₄ alkyl; R₂ and R₃ are each independently selected from linear, branched or cyclic C₁₋₁₀ hydrocarbon groups; R₅ is selected from linear, branched or cyclic C₁₋₁₀ hydrocarbon groups, and R₅ is not tert-butyl; R'₄ is a Boc-protected natural or unnatural amino acid fragment; R₆ is H or monovalent alkali metal ion or C₁₋₄ alkyl;
0, 1 or more C atoms in R₁-R₅ are substituted with heteroatoms O, S, and N; and/or 0, 1, or more H atoms in R₁-R₆ are substituted with halogen, hydroxyl, alkoxy, cyano, and/or nitro.

Further, R is 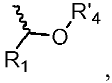 R₁ is H or C₁₋₄ alkyl; R'₄ is a Boc-protected natural or unnatural amino acid fragment, and the method also includes the following deprotection step: hydrogen chloride or trifluoroacetic acid is added and reacted to remove Boc group, obtaining 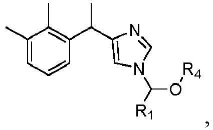 wherein R₄ is a natural or unnatural amino acid fragment.

More further, the solvent in step (1) is at least one of DMF, acetonitrile, or dichloromethane; the acid-binding agent in step (2) is at least one of anhydrous sodium carbonate, anhydrous potassium carbonate, 1,8-diazabicycloundecano-7-ene, triethylamine, and pyridine; and in step (2), a catalyst can also be added, and preferably, it is sodium iodide.

More further, the structure of the compound is as represented by any one of the followings:

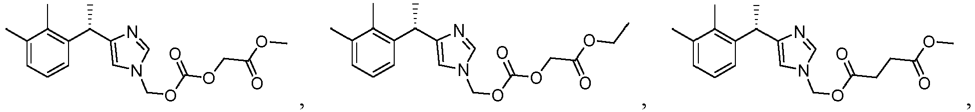

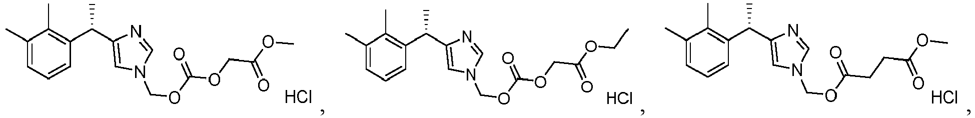

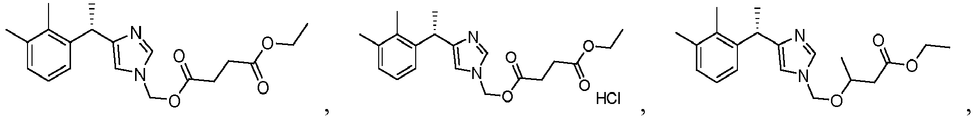

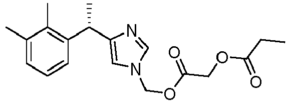

The present invention provides the use of the compound mentioned above, or optical isomers, pharmaceutically acceptable salts, hydrates, or solvates thereof in the manufacture of medicaments that have central sedative, and/or analgesic, and/or hypnotic, and/or anesthetic effects on humans or animals.

The present invention also provides a medicament that causes central sedative, and/or analgesic, and/or hypnotic, and/or anesthetic effects on humans or animals, which is a preparation prepared with the above compound or optical isomers, pharmaceutically acceptable salts, hydrates, or solvates thereof, as the active ingredient, in combination with pharmaceutically acceptable adjuvants, carriers, or excipients.

The results of animal experiments indicate that due to its lack of activity, the prodrug molecule of the present invention does not play an immediate role in the receptor activation, when entering into the body with aid of circulating. Subsequently, after being metabolized by widely distributed hydrolytic enzymes in the body, the parent drug dexmedetomidine is released at an appropriate rate. Animal experiments have shown that at effective doses, one-time intravenous injection of such prodrug molecules not only produces sedative and hypnotic effects, but also does not cause significant changes in blood pressure, and thereby effectively reduces the side effects of hemodynamic changes caused by dexmedetomidine. As a result, the tolerance and safety of the medicament can be improved, and the drug delivery route is simplified.

In animal experiments, the prodrug molecules disclosed in the present patent have similar onset and maintenance times to dexmedetomidine, while significantly reduce hemodynamic changes and make the animal blood pressure relatively stable. Moreover, without the need for continuous administration of 10-15 minutes, these prodrugs can be fully injected into the body within 30 seconds, accompanied by the stable blood pressure. In addition, it is reported that the oral bioavailability of dexmedetomidine is only 16% (Anttila M, et al. Br. J. Clin. Pharmacol. 2003; 56: 691-3). However, the compound according to the present patent can produce a significant sedative effect when administered orally at a certain molar effective dose, while oral administration of equimolar dexmedetomidine cannot produce a sedative effect. The above results indicate that the new compound has a higher oral bioavailability than dexmedetomidine, and thus the compound may have sedative and hypnotic effects by administrating in a non-venous pathway.

Due to the above properties, compounds represented by formula (I) above, and their stereoisomers, isotope-substituted derivatives, pharmaceutically acceptable salts, solvates, pharmaceutical compositions, formulations prepared in combination with pharmaceutically acceptable adjuvents/carriers/excipients, and the like can be used in the manufacture of medicaments that have central sedative, analgesic, and anesthetic effects on humans or animals.

Unless otherwise stated, the terms used in the specification and claims are defined as in the followings.

The carbon, hydrogen, oxygen, sulfur, nitrogen, or halogens involved in the groups and compounds of the present invention all include their isotopes, and are optionally further substituted with one or more corresponding isotopes (i.e. isotope-substituted compounds), wherein the isotopes of carbon include ¹²C, ¹³C, and ¹⁴C; the isotopes of hydrogen include protium (H), deuterium (D, also known as heavy hydrogen) and tritium (T, also known as superheavy hydrogen); the isotopes of oxygen include ¹⁶O, ¹⁷O, and ¹⁸O; the isotopes of sulfur include ³²S, ³³S, ³⁴S, and ³⁶S; the isotopes of nitrogen include ¹⁴N and 1⁵N; the isotopes of fluorine include ¹⁹F; the isotopes of chlorine include ³⁵Cl and ³⁷Cl; and the isotopes of bromine include ⁷⁹Br and ⁸¹Br.

The term "hydrocarbon group" refers to a linear, branched, or cyclic monovalent substituent consisting solely of carbons and hydrogens, which can be a linear or branched or cyclic alkyl, alkenyl, or alkynyl, including phenyl. C₁₋₁₀ hydrocarbon group refers to the number of C atoms in the hydrocarbon group is 1, 2, 3, 4, 5, 6, 7, 8, 9 or 10.

The term "alkyl" refers to a saturated monovalent hydrocarbon group which is straight or branched or cyclic. C₁₋₄ alkyl means the number of C atoms in an alkyl is 1, 2, 3, or 4; Examples of alkyls include but are not limited to methyl, ethyl, n-propyl, isopropyl, n-butyl, isobutyl, sec-butyl, tert-butyl, n-pentyl, 2-pentyl, 3-pentyl, 2-methyl-2-butyl, 3-methyl-2-butyl, n-hexyl, n-heptyl, n-octyl, n-nonyl, and n-decyl.

The term "alkoxy" refers to a monovalent group with a structure of O-alkyl, wherein the alkyl is as defined herein. C₁₋₃ alkoxy means the number of C atoms in the alkyl is 1, 2, or 3. Examples of alkoxy include but are not limited to methoxy, ethoxy, 1-propoxy, 2-propoxy, 1-butoxy, 2-methyl-1-propoxy, 2-butoxy, 2-methyl-2-propoxy, 1-pentoxy, 2-pentoxy, 3-pentoxy, 2-methyl-2-butoxy, 3-methyl-2-butoxy, 3-methyl-1-butoxy, 2-methyl-1-butoxy, and the same. The alkoxy mentioned herein are as defined above.

The term 'cycloalkyl' refers to a monovalent saturated carboncyclic hydrocarbon group, typically having 3-10 carbon atoms. Non-limiting examples include cyclopropyl, cyclobutyl, cyclopentyl, cyclohexyl, or cycloheptyl.

The term "a chemically stable group formed by substituting C atoms in a certain group with heteroatoms O, S, N " means, for example, the carbon C in a methylene -CH₂- of the group structure is substituted with O to form a stable -O-, or is substituted with N to form a stable -NH-, or is substituted with S to form a stable -S- or -SO₂-; alternatively, the group is phenyl, and a C in the phenyl is substituted with N, forming a stable

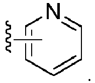

The term "pharmaceutical composition" refers to a mixture of one or more compounds described herein or physiologically/pharmaceutically acceptable salts and other components, wherein other components include physiologically/pharmaceutically acceptable carriers and excipients.

The term "carrier" refers to a carrier or diluent that cannot cause significant stimulation to the organism, and cannot eliminate the biological activity and properties of the compound administrated.

The term "excipient" refers to an inert substance added to a pharmaceutical composition to further rely on the administration of compounds. Examples of excipients include but are not limited to calcium carbonate, calcium phosphate, various sugars, and different types of starch, cellulose derivatives (including microcrystalline cellulose), gelatin, vegetable oils, polyethylene glycols, diluents, granulating agents, lubricants, adhesives, disintegrants, etc.

The term "stereoisomer" refers to an isomer generated by different spatial arrangements of atoms in a molecule, including cis/trans-isomers, enantiomers, and conformational isomers.

The term "effective dose" refers to the amount of a compound that causes physiological or medical reactions in tissues, systems, or subjects. This amount is that searching for, including the amount of a compound that is sufficient to prevent the occurrence of one or more symptoms of a disorder or condition being treated or to reduce them to a certain extent when administered to the subject.

The term "solvate" means that the compounds of the present invention or salts thereof include stoichiometric or non-stoichiometric solvents that bind with non-covalent intermolecular forces. When the solvent is water, the solvate is a hydrate.

"DMF" is N,N-dimethylformamide.

"Boc" is tert-butoxycarbonyl, used to protect amino groups.

The term "a natural or unnatural amino acid fragment" is a group formed by removal of a partial structure (such as removing H, OH, COOH, or NH₂) from an amino acid.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Examples

The raw materials and equipment used in the present invention are known products obtained by purchasing those commercially available.

### Example 1. Preparation of a compound according to the present invention

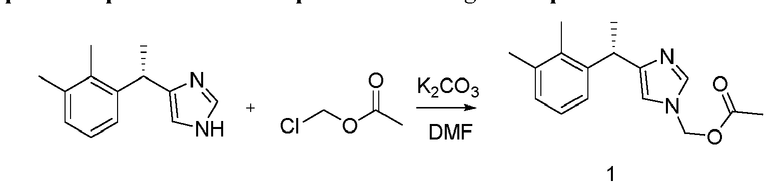

200 mg of dexmedetomidine free base was dissolved in 10 mL of N,N-dimethylformamide (DMF), to which were added 120 mg of ethyl chloroformate and 300 mg of anhydrous potassium carbonate, and then the mixture was stirred overnight at 50 °C. The next day, the reaction solution was cooled, filtered, and the filtrate was evaporated to dryness under reduced pressure, to remove the solvent. After column chromatography (cyclohexane/ethyl acetate = 5/1), 185 mg of compound **1** was obtained as colorless and transparent oily substance, with a yield of 67.8%.

¹H NMR (400 MHz, CDCl₃): *δ* 1.58 (3H, d, *J* = 8 Hz), 2.08 (3H, s), 2.29 (3H, s), 4.37 (1H, q, *J* = 8 Hz), 5.74 (2H, s), 6.62 (1H, s), 7.01-7.08 (3H, m), 7.62 (1H, s).

### Example 2. Preparation of a compound according to the present invention

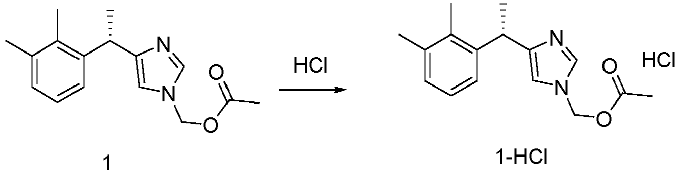

100 mg of compound **1** was dissolved in 10 mL of methanol, to which was added an excess 10% HCl methanol solution (approximately 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **1** (44 mg) was obtained as transparent oil, with a yield of 38.8%.

¹H NMR (400 MHz, CDCl₃): *δ* 1.59 (3H, d, *J=* 8 Hz), 2.05 (3H, s), 2.26 (3H, s), 4.41 (1H, q, *J=* 8 Hz), 5.71 (2H, s), 6.69 (1H, s), 7.02-7.09 (3H, m), 8.23 (1H, s).

### Example 3. Preparation of a compound according to the present invention

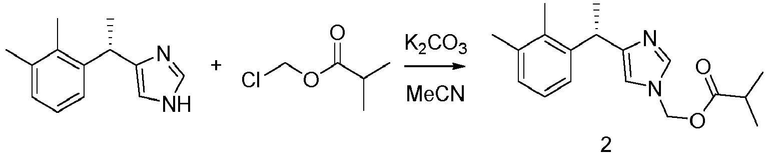

200 mg of dexmedetomidine free base was dissolved in 10 mL of acetonitrile (MeCN), to which were added 204 mg of chloromethyl isobutyrate and 300 mg of anhydrous potassium carbonate, and then the mixture was stirred overnight at 50 °C. The next day, the reaction solution was cooled, filtered, and the filtrate was evaporated to dryness under reduced pressure, to remove the solvent. After column chromatography (cyclohexane/ethyl acetate = 5/1), 171 mg of compound **2** was obtained as white solids, with a yield of 57.0%.

¹H NMR (400 MHz, CDCl₃): *δ* 1.14 (6H, d, *J* = 8 Hz), 1.59 (3H, d, *J* = 8 Hz), 2.23 (3H, s), 2.29 (3H, s), 2.54 (1H, m), 4.38 (1H, q, *J* = 8 Hz), 5.76 (2H, s), 6.61 (1H, s), 6.98-7.10 (3H, m), 7.68 (1H, s).

### Example 4. Preparation of a compound according to the present invention

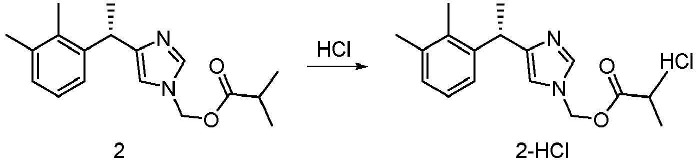

100 mg of compound **2** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **2** (68 mg) was obtained as transparent oil, with a yield of 60.7%.

¹H NMR (400 MHz, CDCl₃): *δ* 1.15 (6H, d, *J* = 8 Hz), 1.72 (3H, d, *J* = 8 Hz), 2.21 (3H, s), 2.30 (3H, s), 2.59 (1H, m), 4.57 (1H, q, *J* = 8 Hz), 6.03 (2H, s), 6.66 (1H, s), 7.03-7.10 (3H, m), 9.18 (1H, s).

### Example 5. Preparation of a compound according to the present invention

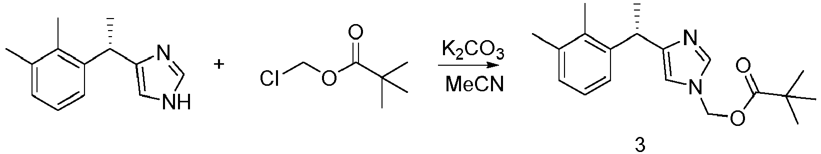

200 mg of dexmedetomidine free base was dissolved in 10 mL of acetonitrile (MeCN), to which were added 225 mg of chloromethyl pivalate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 155 mg of compound **3** was obtained as white solids, with a yield of 49.5%.

¹H NMR (400 MHz, CDCl₃): *δ* 1.16 (3H, s), 1.57 (3H, d, *J* = 8 Hz), 2.23 (3H, s), 2.28 (3H, s), 4.36 (1H, q, *J* = 8 Hz), 5.74 (2H, s), 6.61 (1H, s), 6.99-7.05 (3H, m), 7.59 (1H, s).

### Example 6. Preparation of a compound according to the present invention

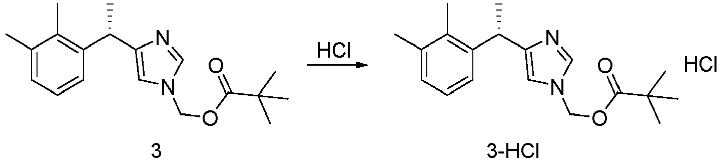

100 mg of compound **3** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound 3 (70 mg) was obtained as transparent oil, with a yield of 62.7%.

¹H NMR (400 MHz, CDCl₃): *δ* 1.17 (3H, s), 1.72 (3H, d, *J* = 8 Hz), 2.20 (3H, s), 2.30 (3H, s), 4.58 (1H, q, *J* = 8 Hz), 6.05 (2H, s), 6.64 (1H, s), 7.03-7.11 (3H, m), 9.26 (1H, s).

### Example 7. Preparation of a compound according to the present invention

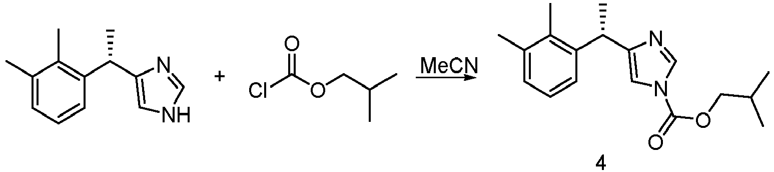

200 mg of dexmedetomidine free base was dissolved in 10 mL of acetonitrile (MeCN), to which was added 204 mg of isobutyl chloroformate, and then the mixture was stirred overnight at room temperature. The next day, the reaction solution was cooled, filtered, and the filtrate was evaporated under reduced pressure to remove the solvent. After column chromatography (cyclohexane/ethyl acetate = 5/1), 158 mg of compound **4** was obtained as white solids, with a yield of 52.6%.

¹H NMR (400 MHz, CDCl₃): *δ* 1.00 (6H, d, *J* = 8 Hz), 1.58 (3H, d, *J* = 8 Hz), 2.08 (1H, m), 2.26 (3H, s), 2.30 (3H, s), 4.15 (2H, d, *J =* 8 Hz), 4.37 (1H, q, *J =* 8 Hz), 6.99 (1H, s), 7.01-7.07 (3H, m), 8.05 (1H, s).

### Example 8. Preparation of a compound according to the present invention

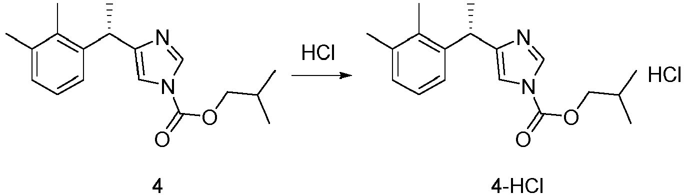

100 mg of compound **4** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **4** (65 mg) was obtained, with a yield of 58%.

¹H NMR (400 MHz, CDCl₃): *δ* 0.94 (6H, d, *J* = 8 Hz), 1.66 (3H, d, *J* = 8 Hz), 2.05 (1H, m), 2.17 (3H, s), 2.24 (3H, s), 4.18 (2H, d, *J* = 8 Hz), 4.51 (1H, q, *J* = 8 Hz), 6.92 (1H, s), 6.98-7.07 (3H, m), 8.60 (1H, s).

### Example 9. Preparation of a compound according to the present invention

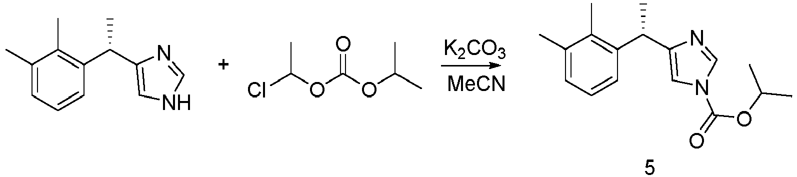

200 mg of dexmedetomidine free base was dissolved in 10 mL of acetonitrile (MeCN), to which was added 227 mg of chloromethyl isopropylcarbonate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 166 mg of compound 5 was obtained as transparent oil, with a yield of 58.0%.

¹H NMR (400 MHz, CDCl₃): *δ* 1.39 (6H, d, *J* = 8 Hz), 1.58 (3H, d, *J* = 8 Hz), 2.25 (3H, s), 2.30 (3H, s), 4.36 (1H, q, *J* = 8 Hz), 5.19 (1H, m), 6.98 (1H, s), 7.01-7.08 (3H, m), 8.05 (1H, s).

### Example 10. Preparation of a compound according to the present invention

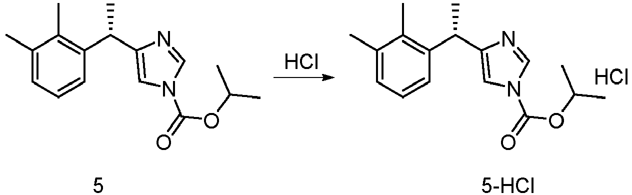

100 mg of compound **5** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **5** (72 mg) was obtained as transparent oil, with a yield of 63.9%.

¹H NMR (400 MHz, CDCl₃): *δ* 1.44 (6H, d, *J* = 8 Hz), 1.69 (3H, d, *J* = 8 Hz), 2.24 (3H, s), 2.31 (3H, s), 4.53 (1H, q, *J* = 8 Hz), 5.26 (1H, m), 6.98 (1H, s), 7.06-7.11 (3H, m), 8.51 (1H, s).

### Example 11. Preparation of a compound according to the present invention

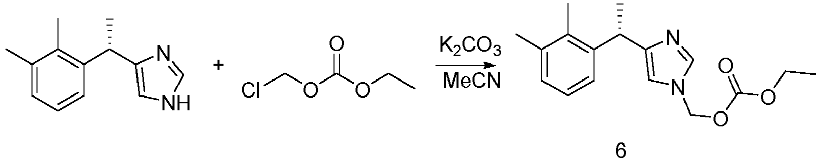

200 mg of dexmedetomidine free base was dissolved in 10 mL of acetonitrile (MeCN), to which was added 227 mg of chloromethyl ethylcarbonate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 171 mg of compound **6** was obtained as white solids, with a yield of 56.4%.

¹H NMR (400 MHz, CDCl₃): *δ* 1.24 (3H, t, *J* = 8 Hz), 1.51 (3H, d, *J* = 8 Hz), 2.17 (3H, s), 2.22 (3H, s), 4.16 (2H, q, *J* = 8 Hz), 4.30 (1H, q, *J* = 8 Hz), 5.68 (2H, s), 6.57 (1H, s), 6.93-7.03 (3H, m), 7.55 (1H, s).

### Example 12. Preparation of a compound according to the present invention

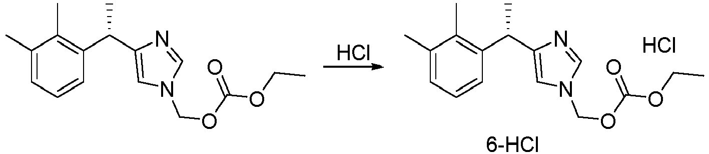

100 mg of compound **6** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **6** (75 mg) was obtained as transparent oil, with a yield of 67%.

¹H NMR (400 MHz, CDCl₃): *δ* 1.25 (3H, t, *J* = 8 Hz), 1.67 (3H, d, *J* = 8 Hz), 2.15 (3H, s), 2.24 (3H, s), 4.18 (2H, q, *J* = 8 Hz), 4.55 (1H, q, *J* = 8 Hz), 6.02 (2H, s), 6.62 (1H, s), 7.00-7.05 (3H, m), 9.34 (1H, s).

### Example 13. Preparation of a compound according to the present invention

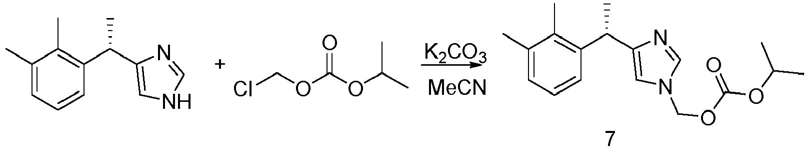

200 mg of dexmedetomidine free base was dissolved in 10 mL of acetonitrile (MeCN), to which was added 228 mg of chloromethyl isopropylcarbonate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 164 mg of compound **7** was obtained as transparent oil, with a yield of 51.9%.

¹H NMR (400 MHz, CDCl₃): *δ* 1.30 (6H, d, *J* = 8 Hz), 1.58 (3H, d, *J* = 8 Hz), 2.24 (3H, s), 2.29 (3H, s), 4.37 (1H, q, *J* = 8 Hz), 4.89 (1H, m), 5.74 (2H, s), 6.64 (1H, s), 6.99-7.10 (3H, m), 7.62 (1H, s).

### Example 14. Preparation of a compound according to the present invention

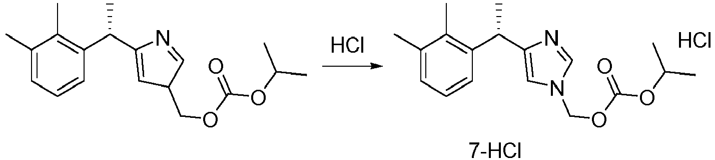

100 mg of compound **7** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **7** (65 mg) was obtained as transparent oil, with a yield of 58.1%.

¹H NMR (400 MHz, CDCl₃): *δ* 1.30 (6H, d, *J* = 8 Hz), 1.63 (3H, d, *J* = 8 Hz), 2.23 (3H, s), 2.29 (3H, s), 4.45 (1H, q, *J* = 8 Hz), 4.90 (1H, m), 5.82 (2H, s), 6.64 (1H, s), 7.01-7.11 (3H, m), 9.13 (1H, s).

### Example 15. Preparation of a compound according to the present invention

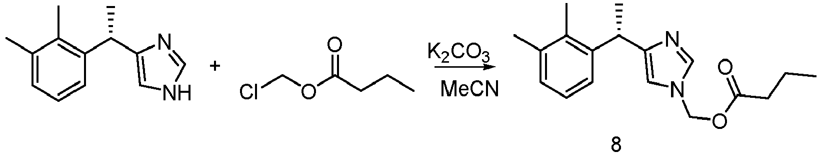

200 mg of dexmedetomidine free base was dissolved in 10 mL of acetonitrile (MeCN), to which was added 204 mg of chloromethyl butyrate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 145 mg of compound **8** was obtained as transparent oil, with a yield of 48.3%.

¹H NMR (400 MHz, CDCl₃): *δ* 0.90 (3H, t, *J* = 8 Hz), 1.52-1.69 (5H, m), 2.24 (3H, s), 2.27-2.34 (5H, m), 4.38 (1H, q, *J* = 8 Hz), 5.75 (2H, s), 6.62 (1H, s), 6.99-7.09 (3H, m), 7.67 (1H, s).

### Example 16. Preparation of a compound according to the present invention

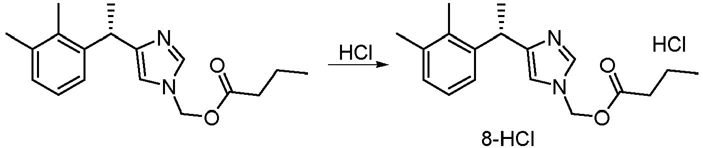

100 mg of compound **8** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **8** (68 mg) was obtained as transparent oil, with a yield of 60.7%.

¹H NMR (400 MHz, DMSO): *δ* 0.85 (3H, t, *J* = 8 Hz), 1.49-1.59 (5H, m), 2.22 (3H, s), 2.26 (3H, s), 2.38 (2H, t, *J* = 8 Hz), 4.49 (1H, q, *J* = 8 Hz), 6.07 (2H, s), 6.85 (1H, s), 7.02-7.09 (2H, m), 7.61 (1H, s), 9.24 (1H, s).

### Example 17. Preparation of a compound according to the present invention

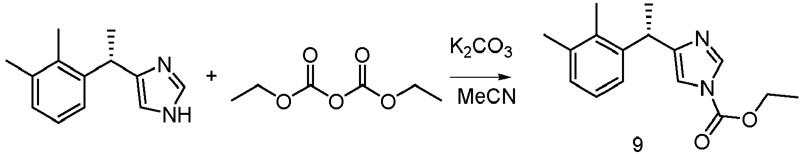

200 mg of dexmedetomidine free base was dissolved in 10 mL of acetonitrile (MeCN), to which was added 243 mg of diethyl pyrocarbonate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 5/1), 170 mg of compound 9 was obtained as transparent oil, with a yield of 62.5%.

¹H NMR (400 MHz, CDCl₃): *δ* 1.41 (3H, t, *J* = 8 Hz), 1.58 (3H, d, *J* = 8 Hz), 2.25 (3H, s), 2.30 (3H, s), 4.32-4.47 (4H, m), 6.98 (1H, s), 7.01-7.08 (3H, m), 8.05 (1H, s).

### Example 18. Preparation of a compound according to the present invention

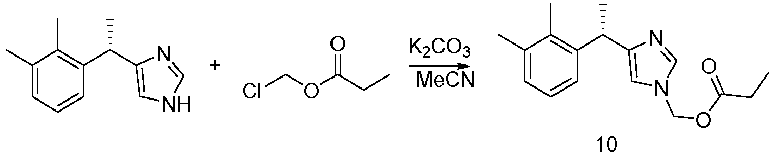

200 mg of dexmedetomidine free base was dissolved in 10 mL of acetonitrile (MeCN), to which was added 240 mg of chloromethyl propionate and 300 mg of anhydrous potassium carbonate, and then the mixture was stirred overnight at 50 °C. The next day, the reaction solution was cooled, filtered, and the filtrate was evaporated under reduced pressure to remove the solvent. After column chromatography (cyclohexane/ethyl acetate = 20/1), 174 mg of compound 10 was obtained as transparent oil, with a yield of 60.9%.

¹H NMR (400 MHz, DMSO): *δ* 0.99 (3H, t, *J* = 8 Hz), 1.44 (3H, d, *J* = 8 Hz), 2.19 (3H, s), 2.23 (3H, s), 2.33 (2H, q, *J* = 8 Hz), 4.25 (1H, q, *J* = 8 Hz), 5.85 (2H, s), 6.86 (1H, s), 6.90-6.99 (3H, m), 7.67 (1H, s).

### Example 19. Preparation of a compound according to the present invention

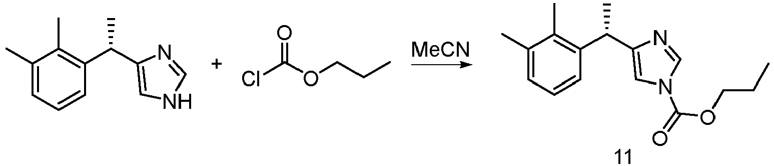

200 mg of dexmedetomidine free base was dissolved in 10 mL of acetonitrile (MeCN), to which was added 183.56 mg of n-propyl chloroformate, and then the mixture was stirred overnight at room temperature. The next day, the reaction solution was cooled, filtered, and the filtrate was evaporated under reduced pressure to remove the solvent. After column chromatography (cyclohexane/ethyl acetate = 50/1), 185 mg of compound **11** was obtained as transparent oil, with a yield of 61.2%.

¹H NMR (400 MHz, DMSO): *δ* 0.94 (3H, t, *J* = 8 Hz), 1.44 (3H, d, *J* = 8 Hz), 1.71 (2H, m), 2.21 (3H, s), 2.23 (3H, s), 4.29 (3H, m), 6.98 (3H, m), 7.18 (1H, s), 8.16 (1H, s).

### Example 20. Preparation of a compound according to the present invention

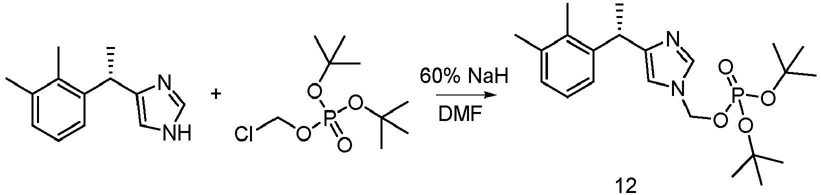

200 mg of dexmedetomidine free base was dissolved in 10 mL of *N,N*-dimethylformamide (DMF), to which was added 139.81 mg of 60% NaH, and then the reaction was stirred vigorously at room temperature for 45 min. Then, 335.81 mg of di-tert-butyl chloromethyl phosphate (dissolved in 5 mL of DMF) was slowly added, and the reaction was stirred overnight at room temperature. The next day, the reaction solution was cooled, filtered, and the filtrate was evaporated under reduced pressure to remove the solvent. After column chromatography (cyclohexane/ethyl acetate = 5/1), 170 mg of compound **12** was obtained as transparent oil, with a yield of 40.38%.

¹H NMR (400 MHz, DMSO): *δ* 1.32 (18H, s), 1.44 (3H, d, *J* = 8 Hz), 2.18 (3H, s), 2.23 (3H, s), 4.26 (1H, q, *J* = 8 Hz), 5.67 (2H, m), 6.83 (1H, s), 6.90-7.08 (3H, m), 7.71 (1H, s).

### Example 21. Preparation of a compound according to the present invention

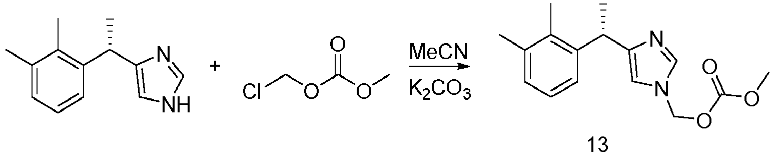

200 mg of dexmedetomidine free base was dissolved in 10 mL of acetonitrile (MeCN), to which was added 248.68 mg of chloromethyl methyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 182 mg of compound **13** was obtained as transparent oil, with a yield of 63.2%.

¹H NMR (400 MHz, DMSO): *δ* 1.44 (3H, d, *J* = 8 Hz), 2.19 (3H, s), 2.23 (3H, s), 3.72 (3H, s), 4.25 (1H, q, *J* = 8 Hz), 5.89 (2H, s), 6.89 (1H, s), 6.90-6.99 (3H, m), 7.70 (1H, s).

### Example 22. Preparation of a compound according to the present invention

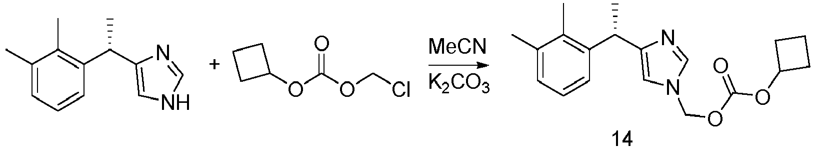

200 mg of dexmedetomidine free base was dissolved in 10 mL of acetonitrile (MeCN), to which was added 328.72 mg of chloromethyl cyclobutyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 195 mg of compound **14** was obtained as transparent oil, with a yield of 59.5%.

¹H NMR (400 MHz, DMSO): *δ* 1.44 (3H, d, *J* = 8 Hz), 1.50-1.61 (1H, m), 1.68-1.77 (1H, m), 1.97-2.06 (2H, m), 2.19 (3H, s), 2.23-2.30 (5H, m), 4.24 (1H, q, *J* = 8 Hz), 4.86 (1H, m), 5.81 (2H, s), 6.88 (1H, s), 6.95-7.01 (3H, m), 7.69 (1H, s).

### Example 23. Preparation of a compound according to the present invention

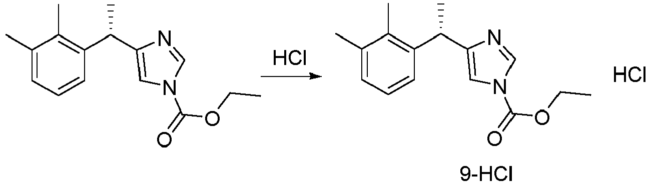

100 mg of compound **9** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **9** (75 mg) was obtained as transparent oil.

¹H NMR (400 MHz, DMSO): *δ* 1.34 (3H, t, *J* = 8 Hz), 1.49-1.59 (5H, m), 2.23 (3H, s), 2.25 (3H, s), 4.25-4.53 (3H, m), 6.07 (2H, s), 6.87-7.10 (3H, m), 7.36 (1H, s), 8.56 (1H, s).

### Example 24. Preparation of a compound according to the present invention

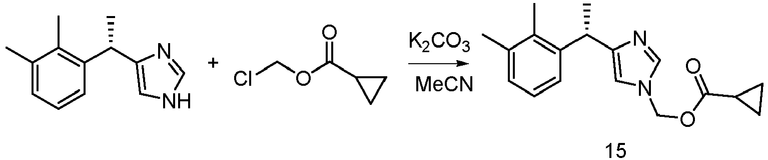

200 mg of dexmedetomidine free base was dissolved in 10 mL of acetonitrile (MeCN), to which was added 201.56 mg of cyclopropyl chloroformate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 189 mg of compound **15** was obtained as transparent oil, with a yield of 63.4%.

¹H NMR (400 MHz, DMSO): *δ* 0.80-0.87 (2H, m), 0.87-0.95 (2H, m), 1.44 (3H, d, *J* = 8 Hz), 1.58-1.68 (1H, m), 2.19 (3H, s), 2.23 (3H, s), 4.24 (1H, q, *J* = 8 Hz), 5.85 (2H, s), 6.86 (1H, s), 6.92-7.00 (3H, m), 7.67 (1H, s).

### Example 25. Preparation of a compound according to the present invention

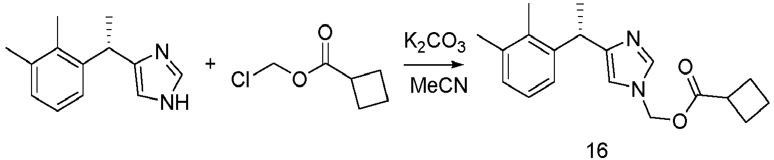

200 mg of dexmedetomidine free base was dissolved in 10 mL of acetonitrile (MeCN), to which was added 222.57 mg of cyclobutyl chloroformate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 201 mg of compound **16** was obtained as transparent oil, with a yield of 64.5%.

¹H NMR (400 MHz, DMSO): *δ* 1.44 (3H, d, *J* = 8 Hz), 1.50-1.62 (1H, m), 1.67-1.77 (1H, m), 1.93-2.07 (2H, m), 2.19 (3H, s), 2.20-2.30 (5H, m), 4.24 (1H, q, *J* = 8 Hz), 4.86 (1H, m), 5.87 (2H, s), 6.88 (1H, s), 6.94-7.02 (3H, m), 7.69 (1H, s).

### Example 26. Preparation of a compound according to the present invention

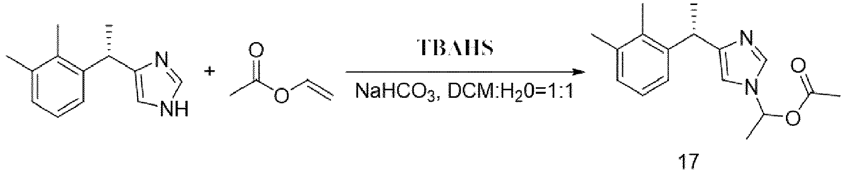

200 mg of dexmedetomidine free base was dissolved in 5 mL of dichloromethane (DCM), to which were added 5 ml of water, 336 mg of sodium bicarbonate, and 34 mg of tetrabutylammonium hydrogen sulfate (TBAHS), and then the reaction was stirred in an ice salt bath for 10 min. Then, 343.88 mg of vinyl acetate was added dropwise, and the reaction was stirred overnight at room temperature. The next day, the reaction solution was cooled, filtered, and the filtrate was evaporated under reduced pressure to remove the solvent. After column chromatography (cyclohexane/ethyl acetate = 20/1), 195 mg of compound **17** was obtained as transparent oil, with a yield of 67.2%.

¹H NMR (400 MHz, DMSO): *δ* 1.44 (3H, d, *J* = 8 Hz), 1.67 (3H, t, *J* = 4 Hz), 2.00 (3H, s), 2.21 (3H, s), 2.23 (3H, s), 4.24 (1H, q, *J* = 8 Hz), 6.64 (1H, q, *J* = 8 Hz), 6.93-7.07 (4H, m), 7.72 (1H, s).

### Example 27. Preparation of a compound according to the present invention

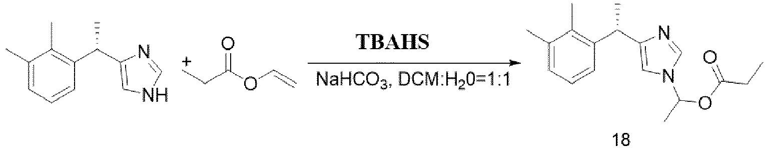

200 mg of dexmedetomidine free base was dissolved in 5 mL of dichloromethane (DCM), to which were added 5 ml of water, 336 mg of sodium bicarbonate, and 34 mg of tetrabutylammonium hydrogen sulfate (TBAHS), and then the reaction was stirred in an ice salt bath for 10 min. Then, 395.88 mg of vinyl propionate was added dropwise, and the reaction was stirred overnight at room temperature. The next day, the reaction solution was cooled, filtered, and the filtrate was evaporated under reduced pressure to remove the solvent. After column chromatography (cyclohexane/ethyl acetate = 20/1), 199 mg of compound **18** was obtained as transparent oil, with a yield of 70. 1%.

¹H NMR (400 MHz, DMSO): *δ* 0.97 (3H, t, *J* = 8 Hz), 1.43 (3H, d, *J* = 8 Hz), 1.67 (3H, t, *J* = 8 Hz), 2.00 (3H, s), 2.20 (3H, s), 2.22 (3H, s), 2.25-2.34 (2H, m), 4.24 (1H, q, *J* = 8 Hz), 6.65 (1H, q, *J* = 8 Hz), 6.91-7.06 (4H, m), 7.72 (1H, s).

### Example 28. Preparation of a compound according to the present invention

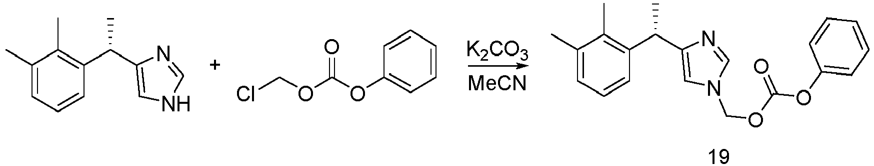

200 mg of dexmedetomidine free base was dissolved in 10 mL of acetonitrile (MeCN), to which was added 279.50 mg of chloromethyl phenyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 210 mg of compound **19** was obtained as transparent oil, with a yield of 60.1%.

¹H NMR (400 MHz, DMSO): *δ* 1.44 (3H, d, *J* = 8 Hz), 2.20 (3H, s), 2.23 (3H, s), 4.27 (1H, q, *J* = 8 Hz), 6.03 (2H, s), 6.87-7.06 (5H, m), 7.17-7.37 (3H, m), 7.48-7.89 (2H, m), 7.75 (1H, s).

### Example 29. Preparation of a compound according to the present invention

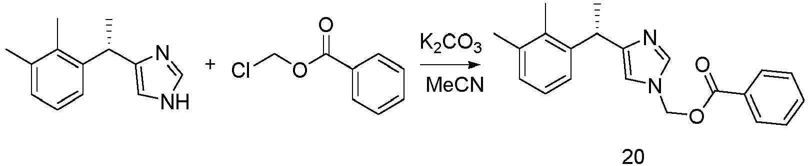

200 mg of dexmedetomidine free base was dissolved in 10 mL of acetonitrile (MeCN), to which was added 255.53 mg of chloromethyl benzoate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 198 mg of compound **20** was obtained as transparent oil, with a yield of 59.3%.

¹H NMR (400 MHz, DMSO): *δ* 1.44 (3H, d, *J* = 8 Hz), 2.19 (3H, s), 2.22 (3H, s), 4.26 (1H, q, *J* = 8 Hz), 6.13 (2H, s), 6.92-7.02 (4H, m), 7.49-7.57 (2H, m), 7.65-7.72 (2H, m), 7.79 (1H, s), 7.91-7.98 (2H, m).

### Example 30. Preparation of a compound according to the present invention

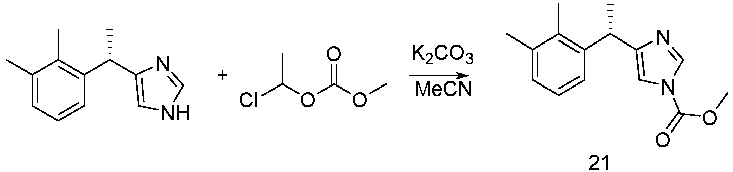

200 mg of dexmedetomidine free base was dissolved in 10 mL of acetonitrile (MeCN), to which was added 207.53 mg of 1-chloroethyl methyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 153 mg of compound **21** was obtained as transparent oil, with a yield of 59.5%.

¹H NMR (400 MHz, DMSO): *δ* 1.46 (3H, d, *J* = 8 Hz), 2.20 (3H, s), 2.23 (3H, s), 3.92 (3H, s), 4.29 (1H, q, *J=* 8 Hz), 6.93-7.01 (3H, m), 7.16 (1H, s), 8.15 (1H, s).

### Example 31. Preparation of a compound according to the present invention

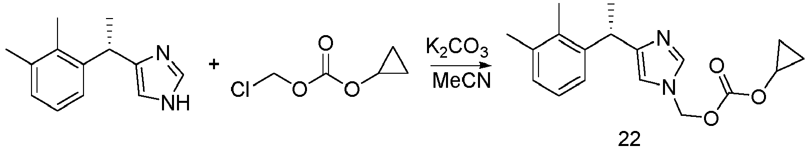

200 mg of dexmedetomidine free base was dissolved in 10 mL of acetonitrile (MeCN), to which was added 225.52 mg of 1-chloromethyl cyclopropyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 20/1), 190 mg of compound **22** was obtained as transparent oil, with a yield of 60.5%.

¹H NMR (400 MHz, DMSO): *δ* 0.57-0.72 (4H, m), 1.44 (3H, d, *J* = 8 Hz), 2.19 (3H, s), 2.23 (3H, s), 4.12 (1H, m), 4.25 (1H, q, *J=* 8 Hz), 5.90 (2H, s), 6.88 (1H, s), 6.94-7.01 (3H, m), 7.70 (1H, s).

### Example 32. Preparation of a compound according to the present invention

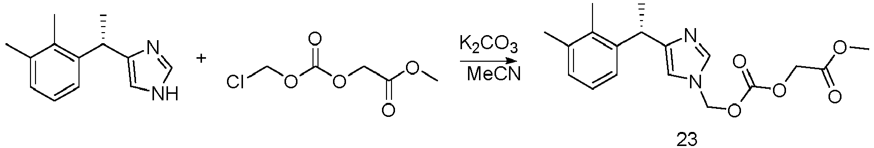

200 mg of dexmedetomidine free base was dissolved in 10 mL of acetonitrile (MeCN), to which was added 273 mg of chloromethyl methoxycarbonylmethyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 6/1), 282.5 mg of compound **23** was obtained as transparent oil, with a yield of 82.7%.

¹H NMR (400 MHz, DMSO-*d₆*): *δ* 1.45 (3H, d, *J* = 8 Hz), 2.20 (3H, s), 2.23 (3H, s), 3.68 (3H, s), 4.25 (1H, q, *J* = 8 Hz), 4.77 (2H, s), 5.96 (2H, s), 6.90 (1H, s), 6.96-7.02 (3H, m), 7.71 (1H, s).

### Example 33. Preparation of a compound according to the present invention

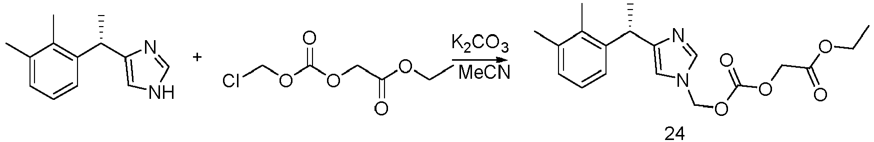

200 mg of dexmedetomidine free base was dissolved in 10 mL of acetonitrile (MeCN), to which was added 229 mg of chloromethyl ethoxycarbonylmethyl carbonate and 300 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 6/1), 254.2 mg of compound **24** was obtained as transparent oil, with a yield of 70.6%.

¹H NMR (400 MHz, DMSO): *δ* 1.17 (3H, t, *J* = 8 Hz), 1.45 (3H, d, *J* = 8 Hz), 2.20 (3H, s), 2.23 (3H, s), 4.13 (2H, q, *J* = 8 Hz), 4.25 (1H, q, *J* = 8 Hz), 4.74 (2H, s), 5.96 (2H, s), 6.90 (1H, s), 6.96-7.02 (3H, m), 7.71 (1H, s).

### Example 34. Preparation of a compound according to the present invention

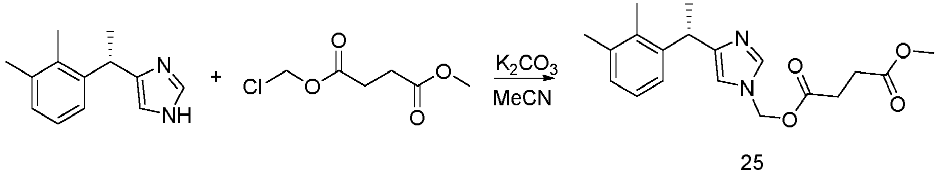

200 mg of dexmedetomidine free base was dissolved in 10 mL of acetonitrile (MeCN), to which was added 229 mg of chloromethyl methyl succinate and 271 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 6/1), 244.3 mg of compound **25** was obtained as transparent oil, with a yield of 81.1%.

¹H NMR (400 MHz, DMSO): *δ* 1.44 (3H, d, *J* = 8 Hz), 2.20 (3H, s), 2.23 (3H, s), 2.53-2.62 (4H, m), 3.55 (3H, s), 4.25 (1H, q, *J* = 8 Hz), 5.88 (2H, s), 6.86 (1H, s), 6.94-7.01 (3H, m), 7.68 (1H, s).

### Example 35. Preparation of a compound according to the present invention

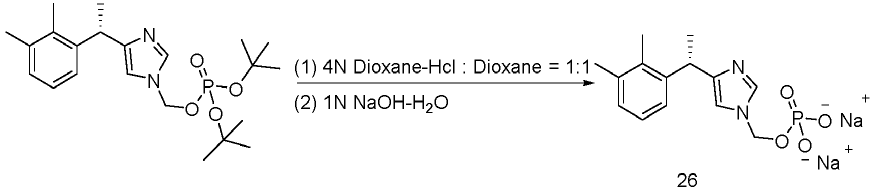

1 g of (*S*)-(4-(1-(2,3-dimethylphenyl)ethyl)-1H-imidazo-1-yl)methyl di-tert-butyl phosphate was dissolved in 10 mL of 4N HCl-dioxane and 10 mL of dioxane, and then the reaction was stirred overnight at room temperature. The reaction solution was rotatory evaporated to dry under reduced pressure, and adjusted to be pH 9 with 1N NaOH solution. Then, 20 mL of acetone was added to precipitate 312 mg of white solids (compound **26**), with a yield of 37.2%.

¹H NMR (400 MHz, D₂O): *δ* 1.44 (3H, d, *J* = 8 Hz), 2.20 (3H, s), 2.23 (3H, s), 4.29 (1H, q, *J* = 8 Hz), 5.40 (2H, d, *J* = 8 Hz), 6.81 (1H, s), 6.92-7.02 (3H, m), 7.64 (1H, s).

### Example 36. Preparation of a compound according to the present invention

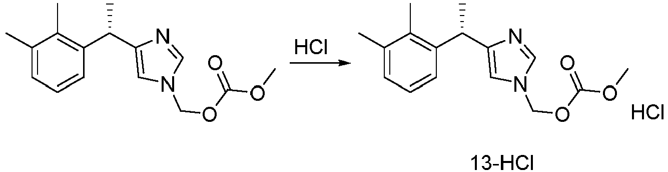

100 mg of compound **13** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **13** (72 mg) was obtained as transparent oil.

¹H NMR (400 MHz, DMSO): *δ* 1.51 (3H, d, *J* = 8 Hz), 2.23 (3H, s), 2.26 (3H, s), 3.77 (3H, s), 4.50 (1H, q, *J* = 8 Hz), 6.00 (2H, s), 6.88 (1H, m), 7.03-7.13 (2H, m), 7.61 (1H, s), 9.23 (1H, s).

### Example 37. Preparation of a compound according to the present invention

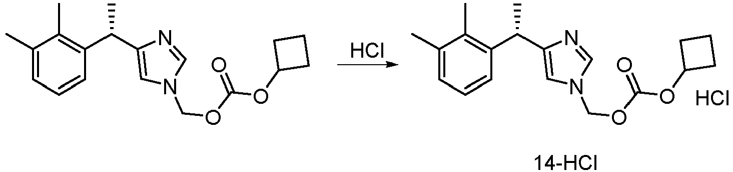

100 mg of compound **14** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **14** (81 mg) was obtained as transparent oil.

¹H NMR (400 MHz, DMSO): *δ* 1.44 (3H, d, *J* = 8 Hz), 1.55 (1H, m), 1.73 (1H, m), 2.01 (2H, m), 2.19 (3H, s), 2.20-2.32 (5H, m), 4.25 (1H, q, *J* = 8 Hz), 4.85 (1H, m), 5.88 (2H, s), 6.91 (1H, s), 6.94-7.02 (3H, m), 7.75 (1H, s).

### Example 38. Preparation of a compound according to the present invention

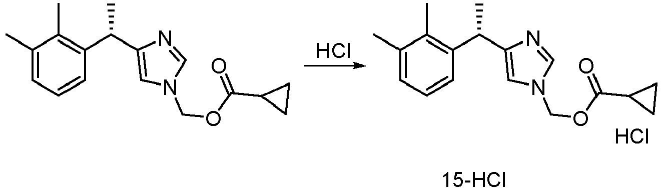

100 mg of compound **15** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **15** (89 mg) was obtained as transparent oil.

¹H NMR (400 MHz, DMSO): *δ* 0.89-0.94 (2H, m), 0.96-1.03 (2H, m), 1.51 (3H, d, *J* = 8 Hz), 1.72 (1H, m), 2.23 (3H, s), 2.26 (3H, s), 4.49 (1H, q, *J* = 8 Hz), 6.05 (2H, s), 6.85 (1H, m), 7.00-7.11 (2H, m), 7.62 (1H, s), 9.19 (1H, s).

### Example 39. Preparation of a compound according to the present invention

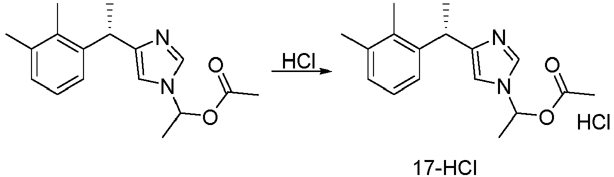

100 mg of compound **17** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/5), the hydrochloride of compound **17** (109 mg) was obtained as transparent oil.

¹H NMR (400 MHz, DMSO): *δ* 1.48 (3H, d, *J* = 8 Hz), 1.74 (3H, t), 2.06 (3H, s), 2.23 (3H, s), 2.25 (3H, s), 4.39 (1H, q, *J* = 8 Hz), 6.72 (1H, q, *J* = 8 Hz), 6.88 (1H, m), 6.97-7.06 (2H, m), 7.52 (1H, d, *J* = 8 Hz), 8.65 (1H, s).

### Example 40. Preparation of a compound according to the present invention

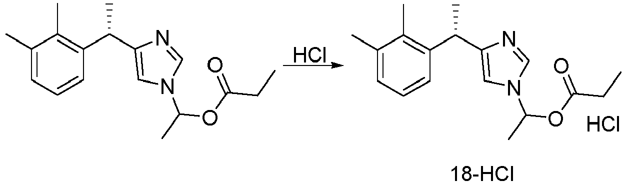

100 mg of compound **18** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **18** (96 mg) was obtained as transparent oil.

¹H NMR (400 MHz, DMSO): *δ* 1.01 (3H, t, *J* = 8 Hz), 1.51 (2H, d, *J* = 8 Hz), 1.79 (3H, t, *J* = 8 Hz), 2.24 (3H, s), 2.26 (3H, s), 2.36-2.43 (2H, m), 4.50 (1H, q, *J* = 8 Hz), 6.76-6.88 (2H, m), 6.97-7.10 (2H, m), 7.52 (1H, d, *J* = 6 Hz), 9.32 (1H, s).

### Example 41. Preparation of a compound according to the present invention

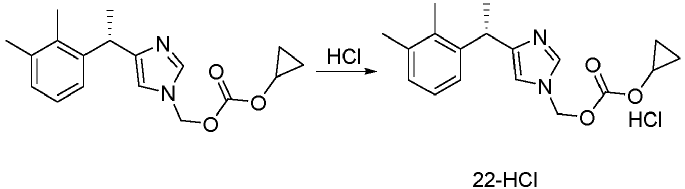

100 mg of compound **22** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **22** (104 mg) was obtained as transparent oil.

¹H NMR (400 MHz, DMSO): *δ* 0.69-0.74 (4H, m), 1.51(3H, d, *J* = 8 Hz), 2.23 (3H, s), 2.26 (3H, s), 2.36-2.43 (2H, m), 4.17 (1H, m), 4.51 (1H, q, *J* = 8 Hz), 6.08 (2H, s), 6.87 (1H, m), 7.01-7.10 (2H, m), 7.60 (1H, s), 9.22 (1H, s).

### Example 42. Preparation of a compound according to the present invention

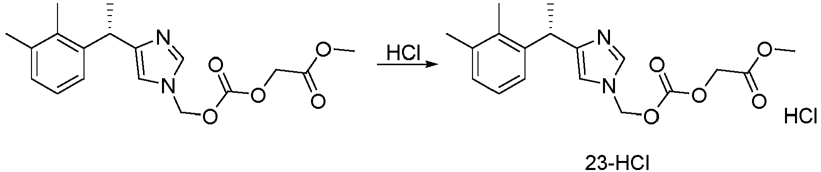

100 mg of compound **23** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **23** (102 mg) was obtained as transparent oil.

¹H NMR (400 MHz, DMSO): *δ* 1.51 (3H, d, *J* = 8 Hz), 2.23 (3H, s), 2.26 (3H, s), 3.68 (3H, s), 4.51 (1H, q, *J* = 8 Hz), 4.82 (2H, s), 6.15 (2H, s), 6.88 (1H, m), 6.99-7.11 (3H, m), 7.63 (1H, s), 9.24 (1H, s).

### Example 43. Preparation of a compound according to the present invention

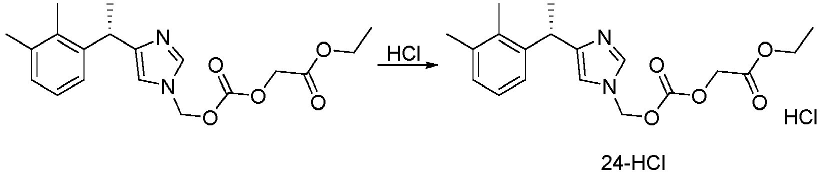

100 mg of compound **24** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **24** (112 mg) was obtained as transparent oil.

¹H NMR (400 MHz, DMSO): *δ* 1.18 (3H, t, *J* = 8 Hz), 1.51 (3H, d, *J* = 8 Hz), 2.23 (3H, s), 2.26 (3H, s), 4.15 (2H, q, *J* = 8 Hz), 4.50 (1H, q, *J* = 8 Hz), 4.80 (2H, s), 6.15 (2H, s), 6.86 (1H, m), 7.01-7.11 (2H, m), 7.63 (1H, s), 9.21 (1H, s).

### Example 44. Preparation of a compound according to the present invention

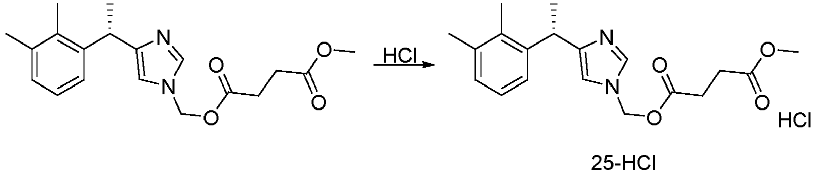

100 mg of compound **25** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **25** (101 mg) was obtained as transparent oil.

¹H NMR (400 MHz, DMSO): *δ* 1.51 (3H, d, *J* = 8 Hz), 2.23 (3H, s), 2.26 (3H, s), 2.57-2.68 (4H, m), 3.56 (3H, s), 4.49 (1H, q, *J* = 8 Hz), 6.08 (2H, s), 6.86 (1H, m), 7.01-7.11 (2H, m), 7.58 (1H, s), 9.17 (1H, s).

### Example 45. Preparation of a compound according to the present invention

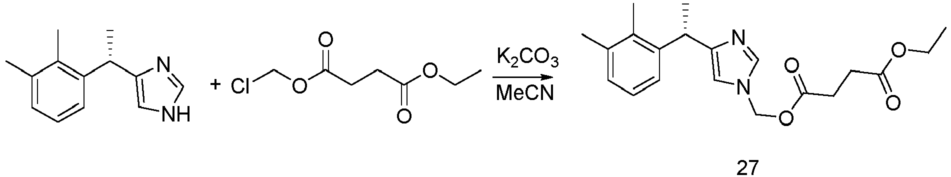

200 mg of dexmedetomidine free base was dissolved in 10 mL of acetonitrile (MeCN), to which was added 292 mg of chloromethyl ethyl succinate and 271 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 10/1), 258.9 mg of compound **27** was obtained as transparent oil, with a yield of 72.33%.

¹H NMR (400 MHz, DMSO): *δ* 1.13 (3H, t, *J* = 8 Hz), 1.43 (3H, d, *J* = 8 Hz), 2.19 (3H, s), 2.23 (3H, s), 2.51-2.62 (4H, m), 4.00 (2H, q, *J* = 8 Hz), 4.25 (1H, q, *J* = 8 Hz), 5.88 (2H, s), 6.86 (1H, m), 6.93-7.01 (3H, m), 7.67 (1H, s).

### Example 46. Preparation of a compound according to the present invention

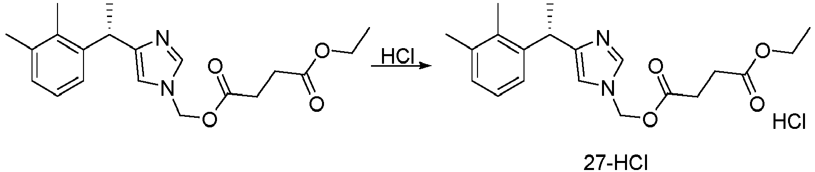

100 mg of compound **27** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **27** (106 mg) was obtained as transparent oil.

¹H NMR (400 MHz, DMSO): *δ* 1.15 (3H, t, *J* = 8 Hz), 1.51 (3H, d, *J* = 8 Hz), 2.23 (3H, s), 2.26 (3H, s), 2.55-2.71 (4H, m), 4.02 (2H, q, *J* = 8 Hz), 4.49 (1H, q, *J* = 8 Hz), 6.09 (2H, s), 6.85 (1H, m), 7.01-7.10 (2H, m), 7.58 (1H, s), 9.16 (1H, s).

### Example 47. Preparation of a compound according to the present invention

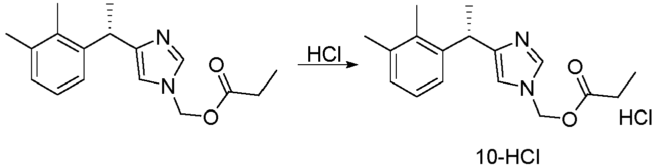

100 mg of compound **10** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **10** (111 mg) was obtained as transparent oil.

¹H NMR (400 MHz, DMSO): *δ* 1.07 (3H, t, *J* = 8 Hz), 1.51 (3H, d, *J* = 8 Hz), 2.23 (3H, s), 2.26 (3H, s), 2.40 (2H, q, *J* = 8 Hz), 4.31 (1H, q, *J* = 8 Hz), 5.92 (2H, s), 6.93 (1H, m), 7.01-7.10 (2H, m), 7.74 (1H, s), 9.16 (1H, s).

### Example 48. Preparation of a compound according to the present invention

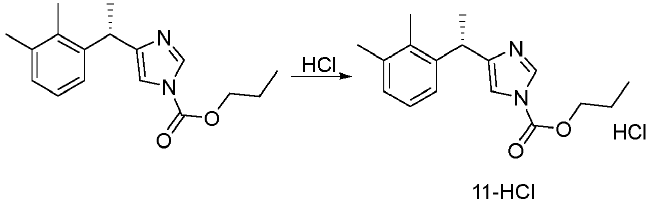

100 mg of compound **11** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **11** (103 mg) was obtained as white solids.

¹H NMR (400 MHz, DMSO): *δ* 0.98 (3H, t, *J* = 8 Hz), 1.51 (3H, d, *J* = 8 Hz), 1.76 (2H, m), 2.23 (3H, s), 2.26 (3H, s), 4.33-4.48 (3H, m), 6.92 (1H, m), 6.97-7.08 (2H, m), 7.62 (1H, s), 9.09 (1H, s).

### Example 49. Preparation of a compound according to the present invention

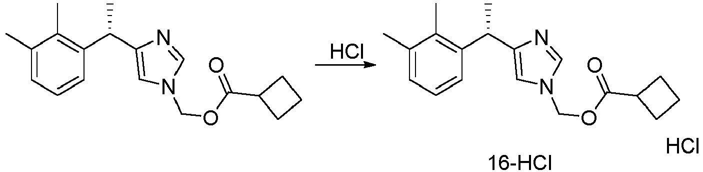

100 mg of compound **16** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **16** (107 mg) was obtained as transparent oil.

¹H NMR (400 MHz, DMSO): *δ* 1.51 (3H, d, *J* = 8 Hz), 1.58 (1H, m), 1.75 (1H, m), 2.05 (2H, m), 2.23 (3H, s), 2.25-2.33 (5H, m), 4.50 (1H, q, *J* = 8 Hz), 4.90 (1H, m), 6.08 (2H, s), 6.86 (1H, m), 7.02-7.10 (2H, m), 7.60 (1H, s), 9.18 (1H, s).

### Example 50. Preparation of a compound according to the present invention

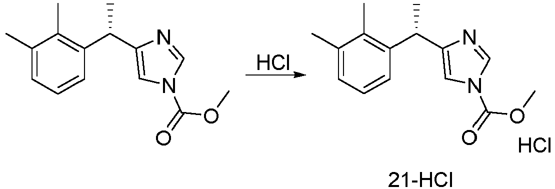

100 mg of compound **21** was dissolved in 10 mL of methanol, to which was added an excess 10% HCl methanol solution (about 1 mL) dropwise, and then the mixture was stirred at room temperature for 10 min. The reaction solution was evaporated to dry under reduced pressure to remove the solvent, and after column chromatography (cyclohexane/ethyl acetate = 1/6), the hydrochloride of compound **21** (105 mg) was obtained as white powder.

¹H NMR (400 MHz, DMSO): *δ* 1.49 (3H, d, *J* = 8 Hz), 2.23 (3H, s), 2.25 (3H, s), 4.00 (3H, s), 4.40 (1H, q, *J* = 8 Hz), 6.08 (2H, s), 6.91 (1H, m), 6.99-7.06 (2H, m), 7.56 (1H, s), 8.98 (1H, s).

### Example 51. Preparation of a compound according to the present invention

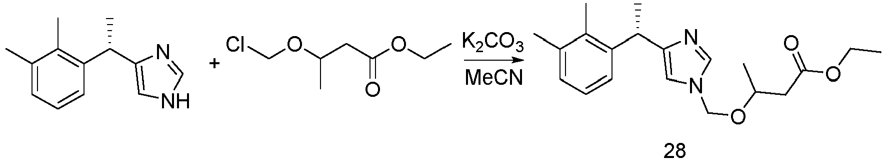

200 mg of dexmedetomidine free base was dissolved in 10 mL of acetonitrile (MeCN), to which was added 271 mg of ethyl 3-(chloromethoxy)butyrate and 271 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 10/1), 245.4 mg of compound **28** was obtained as transparent oil, with a yield of 71.34%.

¹H NMR (400 MHz, DMSO): *δ* 1.11 (3H, t, *J* = 8 Hz), 1.24 (3H, t, *J* = 8 Hz), 1.44 (3H, d, *J* = 8 Hz), 2.19 (3H, s), 2.23 (3H, s), 2.63 (2H, m), 4.02 (2H, m), 4.24 (1H, q, *J* = 8 Hz), 5.00 (1H, m), 5.88 (2H, s), 6.86 (1H, m), 6.94-7.01 (3H, m), 7.69 (1H, s).

### Example 52. Preparation of a compound according to the present invention

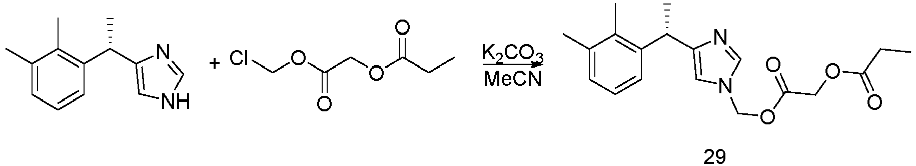

200 mg of dexmedetomidine free base was dissolved in 10 mL of acetonitrile (MeCN), to which was added 271 mg of 2-(chloromethoxy)-2-oxoethyl propionate and 271 mg of anhydrous potassium carbonate, and then the mixture was stirred overnight at room temperature. The next day, the reaction solution was cooled, filtered, and the filtrate was evaporated under reduced pressure to remove the solvent. After column chromatography (cyclohexane/ethyl acetate = 10/1), 243.0 mg of compound **29** was obtained as transparent oil, with a yield of 70.65%.

¹H NMR (400 MHz, DMSO): *δ* 1.03 (3H, t, *J* = 8 Hz), 1.44 (3H, d, *J* = 8 Hz), 2.19 (3H, s), 2.23 (3H, s), 2.39 (2H, q, *J=* 8 Hz), 4.24 (1H, q, *J=* 8 Hz), 4.69 (2H, s), 5.95 (2H, s), 6.86 (1H, m), 6.93-7.03 (3H, m), 7.69 (1H, s).

The beneficial effects of the compound according to the present invention were demonstrated with reference to the following Experimental examples.

### Experimental example 1: Degradation of compounds according to the present invention

### 1. Experimental methods

The test prodrug was formulated as a 1 mg/mL of physiological saline solution, and then 100 µL of the prodrug solution was added to 900 µL of rat plasma. The test solution was vortexed for 30 s before incubation at 37 °C. 50 µL of drug-containing plasma was collected at 0.5 min, 1 min, 2 min, 3 min, 5 min, 10 min, 15 min, 20 min, 30 min, and 60 min, respectively, to which was immediately added 150 µL of acetonitrile to terminate the enzymatic reaction, followed by adding the solution of internal standard telmisartan in methanol (30 µL, 20 µg/mL). Then, the resultant solution was centrifuged at 20000 rpm and 4 °C for 10 min. 100 µL of the supernatant was injected for detection by HPLC, and the concentration of dexmedetomidine was determined by an internal standard method. The degradation rate of the prodrug was calculated based on the concentration of dexmedetomidine. The chromatographic conditions are: Swell XDB C18 column (150 mm × 4.6 mm, 5 µm); column temperature 30 °C; using gradient elution (see Table 1), mobile phase A: methanol, mobile phase B: 0.1% trifluoroacetic acid in water, and mobile phase C: acetonitrile; detection wavelength: 220 nm; flow rate: 1.0 mL/min; injection volume: 30 µL; retention time: 4.7 minutes for dexmedetomidine and 6.7 minutes for internal standard telmisartan. The linear range of dexmedetomidine: 2.5-100 µg/mL. Instruments: Waters 2695 high-performance liquid chromatograph, Waters 2996 photodiode array detector.

**Table 1. Gradient elution program.**

| Time (time)/min | Mobile phase A /% | Mobile phase B /% | Mobile phase C /% |
|---|---|---|---|
| 0 | 20 | 60 | 20 |
| 3 | 20 | 60 | 20 |
| 7 | 10 | 20 | 70 |
| 9 | 10 | 20 | 70 |
| 9.5 | 20 | 60 | 20 |
| 13 | 20 | 60 | 20 |

### 2. Experimental results

The content of dexmedetomidine in plasma samples at various time points was detected, and the degradation rate of prodrug was calculated. The results are shown in Table 2.

**Table 2. The degradation rate of prodrugs.**

| Compound | Degradation rate of prodrug % | | | | | | | | | |
|---|---|---|---|---|---|---|---|---|---|---|
| | 0.5min | 1min | 2 min | 3 min | 5min | 10min | 15 min | 20min | 30min | 60min |
| **1-HCl** | 33 | 68 | 95 | 100 | - | - | - | - | - | - |
| **2-HCl** | 35 | 57 | 87 | 94 | 100 | - | - | - | - | - |
| **3-HCl** | 36 | 73 | 87 | 96 | 100 | - | - | - | - | - |
| **4-HCl** | 31 | 86 | 97 | 100 | 100 | - | - | - | - | - |
| **5-HCl** | 36 | 47 | 52 | 67 | 89 | 98 | 100 | - | - | - |
| **6-HCl** | 21 | 47 | 76 | 89 | 96 | 100 | - | - | - | - |
| **7-HCl** | 22 | 46 | 67 | 88 | 97 | 100 | - | - | - | - |
| **8-HCl** | 76 | 89 | 97 | 100 | - | - | - | - | - | - |
| **9- HCl** | 25 | 44 | 66 | 79 | 87 | 90 | 92 | 96 | 98 | 100 |
| **10-HCl** | 57 | 82 | 85 | 97 | 100 | - | - | - | - | - |
| **11-HCl** | 26 | 57 | 74 | 75 | 82 | 88 | 95 | 100 | - | - |
| **13-HCl** | 16 | 44 | 67 | 79 | 90 | 96 | 100 | - | - | - |
| **14-HCl** | 53 | 80 | 85 | 98 | 100 | - | - | - | - | - |
| **15-HCl** | 18 | 41 | 54 | 63 | 87 | 94 | 100 | - | - | - |
| **16-HCl** | 47 | 78 | 89 | 95 | 100 | - | - | - | - | - |
| **17-HCl** | 12 | 16 | 19 | 22 | 29 | 39 | 48 | 53 | 58 | 67 |
| **18-HCl** | 17 | 33 | 42 | 48 | 54 | 59 | 64 | 69 | 72 | 75 |
| **21-HCl** | 28 | 48 | 59 | 66 | 89 | 98 | 100 | - | - | - |
| **22-HCl** | 91 | 97 | 100 | - | - | - | - | - | - | - |
| **23-HCl** | 23 | - | - | - | 25 | 27 | 28 | 29 | 31 | 34 |
| **24-HCl** | 18 | 21 | 24 | 27 | 31 | 33 | 36 | 37 | 38 | 41 |
| **25-HCl** | 51 | 56 | 59 | 64 | 67 | 68 | 69 | 72 | 76 | 79 |
| **26** | 29 | 31 | 32 | 33 | 34 | 35 | 37 | 38 | 43 | 56 |
| **27-HCl** | 52 | 58 | 66 | - | 78 | 86 | 97 | 100 | - | - |

| | | | | | | | | | | |
|---|---|---|---|---|---|---|---|---|---|---|
| "-", not detected. | | | | | | | | | | |

Based on the experimental results, it was demonstrated that the prodrug prepared in the present invention could release dexmedetomidine in animal plasma in a relatively short time.

### Experimental example 2: Hypnotic effects of compounds according to the present invention

### 1. Experimental methods

Male SD rats, weighing 250-300 g, were randomly divided into groups, with 10 rats in each group. Each drug was dissolved in physiological saline and then injected into the tail vein, with an injection amount of 2× ED₅₀ (ED₅₀: 50% of the dose for the disappearance of righting reflex in rats). After injection, timing was started until the rats were unable to have autonomous righting reflex, i.e. the righting reflex loss, which was served as a hypnotic indicator; after the righting reflex of the rat recovered on its own, it was considered that the effects of drugs had worn off. The onset times and the duration of hypnosis were recorded.

### 2. Experimental results

The results are shown in Table 3:

**Table 3. The data for hypnotic effect of each compound.**

| Compound | ED₅₀ | Dosage | Onset time (min) | Duration (min) |
|---|---|---|---|---|
| | (µg/kg) | (µg/kg) | | |
| Dexmedetomidine Hydrochloride | 18 | 36 | 1.5±0.5 | 53±11 |
| **1-HCl** | 35 | 70 | 3.9±1.5 | 42±9 |
| **2-HCl** | 43 | 86 | 1.1±0.2 | 63±15 |
| **3-HCl** | 59 | 118 | 2.6±1.1 | 50±10 |
| **4-HCl** | 38 | 76 | 2.3±0.9 | 44±8 |
| **5-HCl** | 37 | 74 | 3.7±1.3 | 61±15 |
| **6-HCl** | 30 | 60 | 2.1±1.1 | 45±8 |
| **7-HCl** | 36 | 72 | 4.6±1.3 | 39±11 |
| **8-HCl** | 24 | 48 | 4.1±2.4 | 31±13 |
| **9- HCl** | 22 | 44 | 3.9±1.2 | 28±5 |
| **10-HCl** | 28 | 56 | 2.3±1.1 | 48±12 |
| **11-HCl** | 26 | 52 | 2.6±0.3 | 47±8 |
| **13-HCl** | 25 | 50 | 4.1±1.3 | 36±7 |
| **14-HCl** | 31 | 62 | 3.9±2.1 | 51±7 |
| **15-HCl** | 30 | 60 | 3.5±1.2 | 43±9 |
| **16-HCl** | 35 | 70 | 3.6±1.3 | 44±8 |
| **17-HCl** | 29 | 58 | 1.8±0.7 | 42±12 |
| **18-HCl** | 36 | 72 | 2.4±0.9 | 41±10 |
| **21-HCl** | 23 | 46 | 3.2±1.3 | 35±6 |
| **22-HCl** | 24 | 48 | 1.5±0.2 | 48±9 |
| **23-HCl** | 51 | 102 | 1.7±0.4 | 61±15 |
| **24-HCl** | 30 | 60 | 1.3±0.4 | 47±7 |
| **25-HCl** | 41 | 82 | 3.1±0.9 | 63±15 |
| **26** | 32 | 64 | 1.7±0.3 | 46±5 |
| **27-HCl** | 19 | 38 | 1.4±0.5 | 52±9 |

The above experimental results indicated that the compounds of the present invention could induce sleep in rats within 5 minutes, and the hypnotic time was similar to that of dexmedetomidine. Thus, the compound had definite and reversible hypnotic effects.

### Experimental example 3: Blood pressure change after administration of the compound according to the present invention

### 1. Experimental methods

Male SD rats weighing 250-300 g were randomly divided into groups, with 10 rats in each group. General anesthesia was maintained by inhaling isoflurane. By intubating via femoral artery, blood pressure was monitored using the BL-420N biological signal collection and analysis system. 10 minutes after blood pressure stabilization, the baseline mean arterial pressure (MAP₀) was measured. Subsequently, the physiological saline solution of each test drug was injected via the tail vein (the dosage of each drug was the same as that in Experiment example 2, with a unified injection volume of 1 mL). Among them, there were two injection ways for dexmedetomidine hydrochloride: one is to fully inject within 15 seconds; the other is to inject 0.1 mL per minute and complete the injection within 10 minutes. All prodrug molecules were injected within 15 seconds. After injection of each drug, the change in blood pressure was continuously recorded for 60 min. Except for physiological saline, all drugs showed a rapid increase in blood pressure in the initial stage after administration, followed by a relatively persistent decrease in blood pressure. The difference MAP_{Δ+} between the baseline mean arterial pressure (MAP₀) and the highest mean arterial pressure (MAPₘₐₓ) during the 60 min measurement time was calculated; the difference MAP_{Δ-} between the baseline mean arterial pressure (MAP₀) and the lowest mean arterial pressure (MAPₘᵢₙ) during the 60 min measurement time was also obtained; both of the difference values were used to calculate the maximum increase rate of mean arterial pressure +Δ% = MAP_{Δ+}/MAP₀ * 100% and the maximum decrease rate of mean arterial pressure -Δ% = MAP_{Δ-}/MAP₀ * 100%, respectively. The maximum increase rate of mean arterial pressure +△% and the maximum decrease rate -△% reflect the degree of blood pressure change that the body experienced after administration. The larger the value, the greater the blood pressure change that the body experienced.

### 2. Experimental results

The results are shown in Table 4:

**Table 4. Blood pressure change after administration of each drug.**

| Compound | +Δ% (The maximum increase rate of blood pressure) | -Δ% (The maximum decrease rate of blood pressure) |
|---|---|---|
| Physiological saline | 5±2 | 10±5 |
| Dexmedetomidine hydrochloride (injection within 15 seconds) | 43±11 | 38±7 |
| Dexmedetomidine hydrochloride (injection within 10 minutes) | *15±5 | 37±8 |
| **1-HCl** | *25±12 | 43±10 |
| **2-HCl** | *19±11 | 32±5 |
| **3-HCl** | *13±6 | 41±8 |
| **4-HCl** | 34±14 | 38±9 |
| **5-HCl** | *18±6 | 35±5 |
| **6-HCl** | *23±9 | 36±6 |
| **7-HCl** | *17±8 | 37±8 |
| **8-HCl** | *18±8 | 31±7 |
| **9- HCl** | *21±6 | 41±9 |
| **10-HCl** | 31±7 | 40±8 |
| **11-HCl** | *25±7 | 41±8 |
| **13-HCl** | *16±8 | 39±7 |
| **14-HCl** | 31±6 | 36±6 |
| **15-HCl** | *21±5 | 36±6 |
| **16-HCl** | *25±7 | 42±4 |
| **17-HCl** | *15±4 | 35±6 |
| **18-HCl** | 29±12 | 34±8 |
| **21-HCl** | *16±9 | 43±10 |
| **22-HCl** | 34±7 | 39±6 |
| **23-HCl** | 29±7 | 35±8 |
| **24-HCl** | *27±12 | 36±7 |
| **25-HCl** | 31±14 | 37±7 |
| **26** | *15±8 | 36±8 |
| **27-HCl** | *25±13 | 41±9 |

| | | |
|---|---|---|
| *: p<0.05, compared with dexmedetomidine hydrochloride group (injection within 15 seconds). | | |

The experimental results showed that the physiological saline group had the smallest change in blood pressure before and after injection. Most of the compounds described in the present invention caused relatively small changes in blood pressure of rats before and after administration. Specifically, the maximum increase rate of blood pressure after administration was similar to that of rats in the slow injection group of dexmedetomidine (injection within 10 minutes), which was much lower than that of rats in the rapid injection group of dexmedetomidine (injection within 15 seconds). During the process of sedation and hypnosis induced by rapid injection (injection within 15 seconds) of dexmedetomidine hydrochloride, the maximum increase rate of blood pressure in the initial stage of administration was relatively high, indicating that the change in blood pressure caused by one-time injection was very significant. The above experiments indicated that the compound of the present invention could be rapidly injected and significantly reduce blood pressure changes caused by rapid administration compared to dexmedetomidine

### Experimental example 4: The sedative and hypnotic effects of the compound according the present invention after oral administration

### 1. Experimental methods

Male SD rats weighing 250-300 g were randomly divided into groups, with 10 rats in each group. Each test drug was administered by gavage, at a dosage of 0.16 µmoL/kg for each drug. After gavage, the disappearance of the righting reflex was used as a sign of the hypnotic effect induced by the drug, and the recovery of the righting reflex was used as a sign of drug failure. The onset time and duration of the drug were observed.

### 2. Experimental results

The results are shown in Table 5:

**Table 5. The sedative and hypnotic effects of each drug after oral administration.**

| Compound | Onset time (min) | Duration (min) |
|---|---|---|
| Dexmedetomidine hydrochloride | - | - |
| 1-HCl | 15±6 | 17±8 |
| 4-HCl | 14±8 | 20±13 |
| 7 | 9±4 | 22±8 |
| 10 | 13±6 | 15±7 |
| 12 | 16±7 | 11±4 |
| 13 | 11±5 | 21±9 |
| 17 | 23±5 | 9±4 |
| 23 | 28±5 | 8±2 |

| | | |
|---|---|---|
| -: Disappearance of righting reflex was not observed. | | |

The experimental results showed that at equimolar doses, the compound described in the present patent could produce a hypnotic effect by oral administration, while dexmedetomidine could not produce a hypnotic effect by oral administration. The results indicated that the compound according to the present invention had a higher oral bioavailability than dexmedetomidine, and thus might have sedative and hypnotic effects by administrating in a non-venous pathway.

In summary, the present invention provided a prodrug molecule of dexmedetomidine, which is a 5-benzylimidazole compound. The 5-benzylimidazole compound provided in the present invention did not play an immediate role in the receptor activation, when entering into the body by circulating. Subsequently, after being metabolized by widely distributed hydrolytic enzymes in the body, the parent drug dexmedetomidine was released at a suitable rate. At effective doses, one-time intravenous injection of 5-benzylimidazole compound according to the present invention could not only produce sedative and hypnotic effects, but also cause significant changes in blood pressure. The compound could be injected into the body in just 15 seconds, and effectively reduce the side effects of hemodynamic changes caused by dexmedetomidine. Thereby, the tolerance and safety of the medicament could be improved, and the drug delivery process was simplified.

The compound described in the present patent could produce a significant sedative effect when administered orally at a certain molar effective dose, while oral administration of equimolar dexmedetomidine could not produce a sedative effect. The results indicated that the new compound had a higher oral bioavailability than dexmedetomidine, and thus the compound could have sedative and hypnotic effects by administrating in a non-venous pathway.

## Claims

1. Compounds represented by formula I or optical isomers, pharmaceutically acceptable salts, hydrates, or solvates thereof: 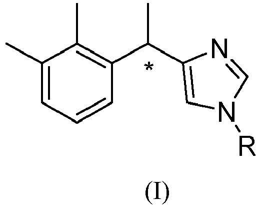
wherein, R is selected from the group consisting of 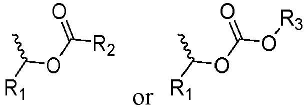 or 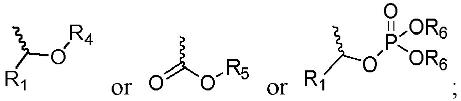
R₁ is selected from the group consisting of H, R^{a}, or R^{b}; R₆ is selected from the group consisting of H, monovalent alkali metal ion, R^{a}, or R^{b};
said R^{a} is substituted or unsubstituted C₁₋₄ alkyl; R^{b} is a chemically stable group formed by substituting some C atoms in R^{a} with heteroatoms O, S, and N;
R₂, R₃, and R₅ are each independently selected from R^{c} or R^{d};
said R^{c} is substituted or unsubstituted C₁₋₁₀ alkyl or 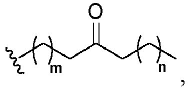 R^{d} is a chemically stable group formed by substituting some C atoms in R^{c} with heteroatoms O, S, and N; and R₅ is not tert-butyl; m is an integer of 0 to 3, and n is an integer of 0 to 3;
R₄ is a natural or unnatural amino acid fragment or 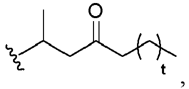 or a chemically stable group formed by substituting a part of C atoms in the natural or unnatural amino acid fragment with heteroatoms O, S, N; t is an integer of 0 to 3;
the substituents are halogen, hydroxyl, alkoxy, cyano, or nitro.

2. The compound according to claim 1 or optical isomers, pharmaceutically acceptable salts, hydrates, or solvates thereof, **characterized in that**:
wherein, R is selected from the group consisting of 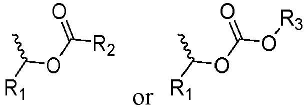 or 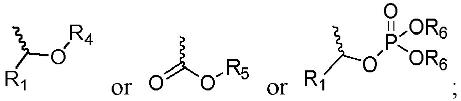
R₁ is selected from the group consisting of H, R^{a} or R^{b}; R₆ is selected from the group consisting of H, monovalent alkali metal ion, R^{a} or R^{b};
said R^{a} is substituted or unsubstituted C₁₋₄ alkyl; R^{b} is a chemically stable group formed by substituting some C atoms in R^{a} with heteroatoms O, S, and N;
R₂, R₃, and R₅ are each independently selected from R^{c} or R^{d};
said R^{c} is substituted or unsubstituted linear, branched or cyclic C₁₋₁₀ hydrocarbon groups; R^{d} is a chemically stable group formed by substituting some C atoms in R^{c} with heteroatoms O, S, and N; and R₅ is not tert-butyl;
R₄ is a natural or unnatural amino acid fragment, or a chemically stable group formed by substituting a part of C atoms in the natural or unnatural amino acid fragment with heteroatoms O, S, N;
the substituents are halogen, hydroxyl, alkoxy, cyano, or nitro.

3. The compound according to claim 2 or optical isomers, pharmaceutically acceptable salts, hydrates, or solvates thereof, **characterized in that** R₁ is H or C₁₋₄ alkyl; R₂ and R₃ are each independently selected from linear, branched or cyclic C₁₋₁₀ hydrocarbon groups; R₅ is selected from linear, branched or cyclic C₁₋₁₀ hydrocarbon groups, and R₅ is not tert-butyl; R₄ is a natural or unnatural amino acid fragment; R₆ is H or monovalent alkali metal ion or C₁₋₄ alkyl;
alternatively, 0, 1 or more C atoms in R₁-R₅ are substituted with heteroatoms O, S, and N to form chemically stable groups; and/or 0, 1, or more H atoms in R₁-R₆ are substituted with halogen, hydroxyl, alkoxy, cyano, and/or nitro.

4. The compound according to claim 2 or optical isomers, pharmaceutically acceptable salts, hydrates, or solvates thereof, **characterized in that** R is 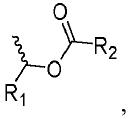 wherein R₁ is H, methyl or ethyl; R₂ is methyl, ethyl, propyl, butyl, isopropyl, cyclopropyl, cyclobutyl, methoxy, ethoxymethyl or vinyl; preferably, the compound has any one of the following structures:

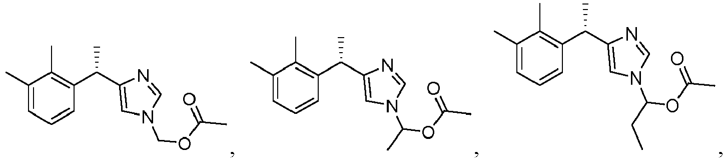

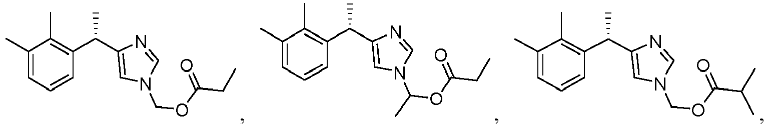

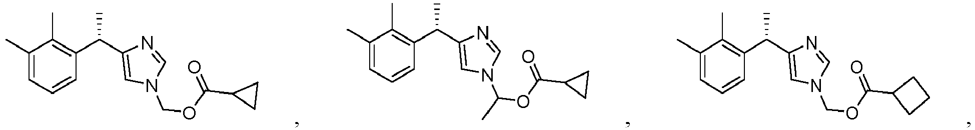

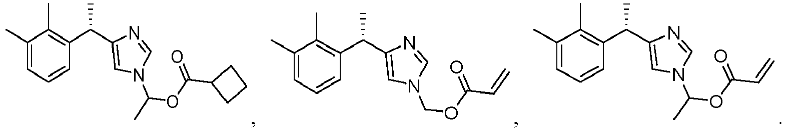

5. The compound according to claim 2 or optical isomers, pharmaceutically acceptable salts, hydrates, or solvates thereof, **characterized in that** R is 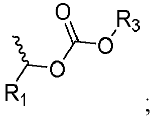 wherein R₁ is H, methyl or ethyl; R₃ is methyl, ethyl, propyl, butyl, isopropyl, cyclopropyl, cyclobutyl, methoxy, ethoxymethyl or vinyl; preferably, the compound has any one of the following structures:

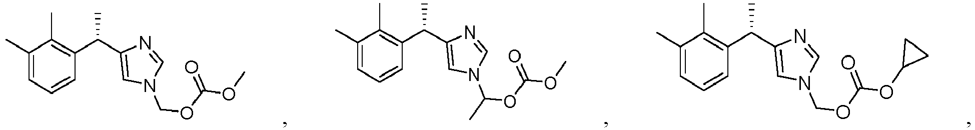

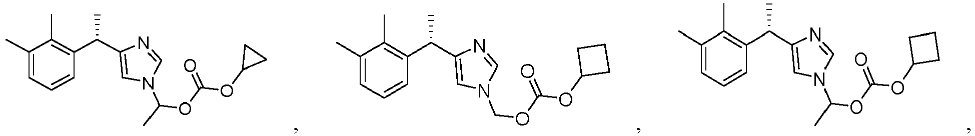

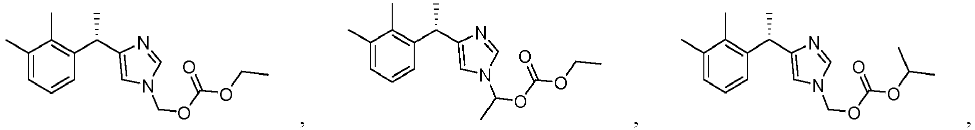

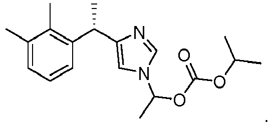

6. The compound according to claim 2 or optical isomers, pharmaceutically acceptable salts, hydrates, or solvates thereof, **characterized in that** R is 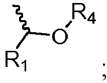 wherein, R₁ is H or methyl; R₄ is glycyl, sarcosinoyl, alanyl, valyl, leucyl, or prolinyl; preferably, the compound has any one of the following structures:

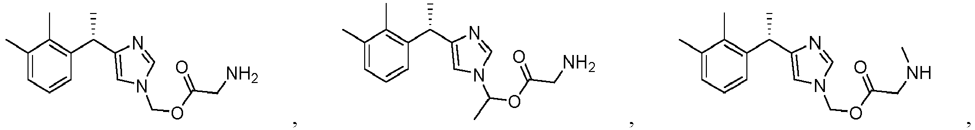

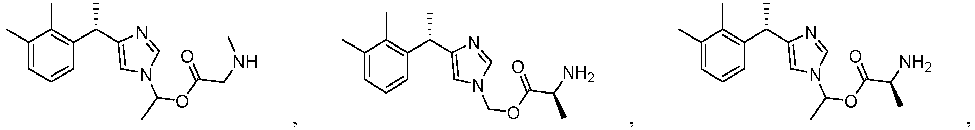

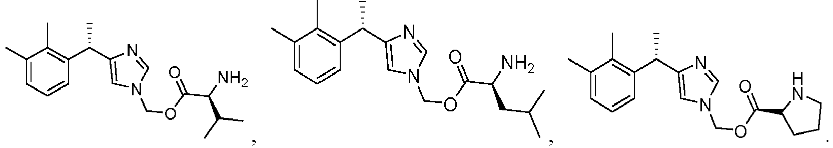

7. The compound according to claim 2 or optical isomers, pharmaceutically acceptable salts, hydrates, or solvates thereof, **characterized in that** R is 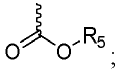 wherein, R₅ is methyl, ethyl, propyl, butyl, isopropyl, isobutyl, cyclopropyl or cyclobutyl; preferably, the compound has any one of the following structures:

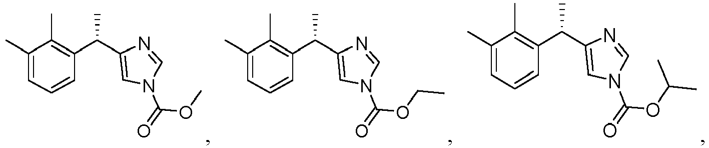

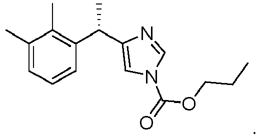

8. The compound according to claim 2 or optical isomers, pharmaceutically acceptable salts, hydrates, or solvates thereof, **characterized in that** R is 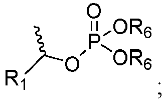 wherein R₆ is methyl, ethyl, propyl, butyl, isopropyl, tert-butyl, isobutyl, cyclopropyl or cyclobutyl; preferably, the compound has any one of the following structures:

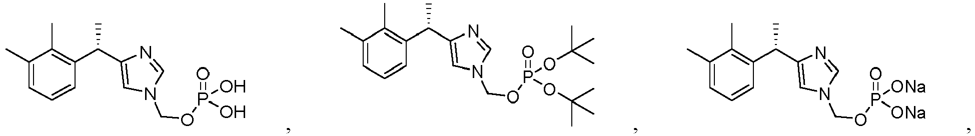

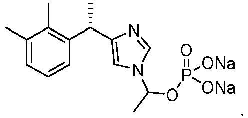

9. The compound according to any one of claims 1 to 8 or optical isomers, pharmaceutically acceptable salts, hydrates, or solvates thereof, **characterized in that** the pharmaceutically acceptable salts include acetate, adipate, alginate, 4-aminosalicylate, ascorbate, aspartate, glutamate, pyroglutamate, benzenesulfonate, benzoate, butyrate, camphorate, camphorsulfonate, carbonate, cinnamate, citrate, cyclamate, cyclopentylpropionate, decanoate, 2,2-dichloroacetate, gluconate, dodecylsulfate, ethane-1,2-disulfonate, ethanesulfonate, formate, fumarate, mucate, trifluoroacetate, gentisate, glucoheptonate, gluconate, glucuronate, glycerophosphate, hydroxy acetate, hemisulfate, heptanoate, caproate, hippurate, hydrochloride, hydrobromate, hydroiodate, 2-hydroxyethanesulfonate, isobutyrate, lactate, lactobionate, laurate, malate, maleate, malonate, mandelate, methanesulfonate, naphthalene-1,5-disulfonate, 2-naphthalenesulfonate, nicotinate, nitrate, octanoate, oleate, orotate, oxalate, 2-oxoglutarate, palmitate, pamoate, pectinate, persulfate, 3-phenylpropionate, phosphate, picrate, neovalerate, propionate, salicylate, sebacate, bisebacate, stearate, succinate, sulfate, tannate, tartrate, bitartrate, thiocyanate, toluenesulfonate or undecanoate, hydrosulfide, sodium salt or ammonium salt of the compounds.

10. The compound according to claim 1 or optical isomers, pharmaceutically acceptable salts, hydrates, or solvates thereof, **characterized in that** the structure of the compound is as represented by any one of the followings:

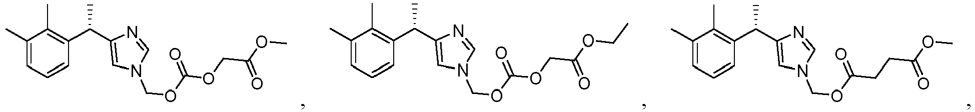

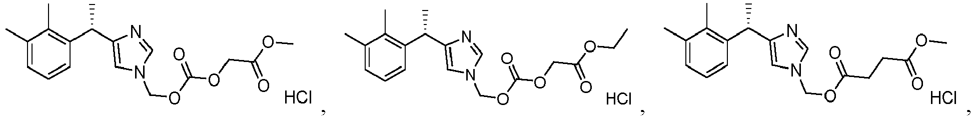

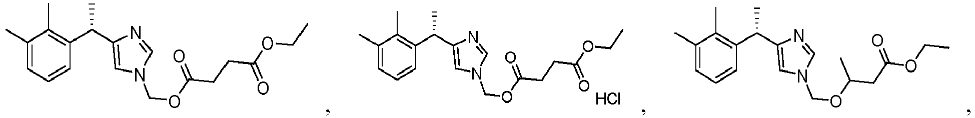

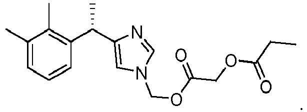

11. A preparation method for the compound according to any one of claims 1 to 9, **characterized in that** it comprises the following steps:
(1) Compound **A** is dissolved in a solvent;
(2) Compound **B** and an acid-binding agent are added and allowed to react;
The reaction scheme is as follows: 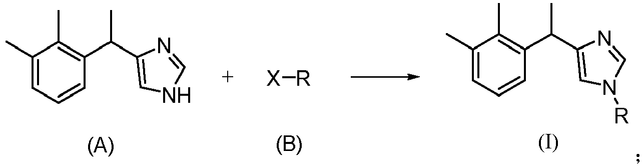
wherein, X is halogen, and preferably Cl;
R is selected from 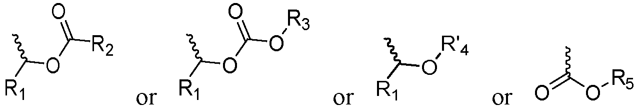 or 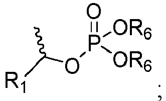
R₁ is H or C₁₋₄ alkyl; R₂ and R₃ are each independently selected from linear, branched or cyclic C₁₋₁₀ hydrocarbon groups; R₅ is selected from linear, branched or cyclic C₁₋₁₀ hydrocarbon groups, and R₅ is not tert-butyl; R'₄ is a Boc-protected natural or unnatural amino acid fragment; R₆ is H or monovalent alkali metal ion or C₁₋₄ alkyl;
0, 1 or more C atoms in R₁-R₅ are substituted with heteroatoms O, S, and N; and/or 0, 1, or more H atoms in R₁-R₆ are substituted with halogen, hydroxyl, alkoxy, cyano, and/or nitro.

12. The preparation method according to claim 11, **characterized in that** R is 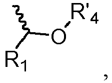 R1 is H or C₁₋₄ alkyl; R'₄ is a Boc-protected natural or unnatural amino acid fragment, and the method also includes the following deprotection step: hydrogen chloride or trifluoroacetic acid is added and reacted to remove Boc group, obtaining 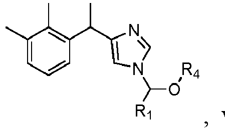 wherein R₄ is a natural or unnatural amino acid fragment.

13. The preparation method according to claim 11 or 12, **characterized in that** the solvent in step (1) is at least one of DMF, acetonitrile, or dichloromethane; the acid-binding agent in step (2) is at least one of anhydrous sodium carbonate, anhydrous potassium carbonate, 1,8-diazabicycloundecano-7-ene, triethylamine, and pyridine; and in step (2), a catalyst can also be added, and preferably, it is sodium iodide.

14. The compound according to any one of claims 1 to 10, or optical isomers, pharmaceutically acceptable salts, hydrates, or solvates thereof, for use in the manufacture of medicaments that have central sedative and/or analgesic and/or hypnotic and/or anesthetic effects on humans or animals.

15. A medicament that causes central sedative and/or analgesic and/or hypnotic and/or anesthetic effects on humans or animals, **characterized in that** it is a preparation prepared with the compound according to any one of claims 1 to 10, or optical isomers, pharmaceutically acceptable salts, hydrates, or solvates thereof, as the active ingredient, in combination with pharmaceutically acceptable adjuvants, carriers, or excipients.
